# FASCICULE DE BREVET EUROPEEN

(11) **EP 0 721 938 B1**
(45) Date de publication et mention de la délivrance du brevet: **08.12.1999**
(21) Numéro de dépôt: 96400051.7
(22) Date de dépôt: 10.01.1996
(51) Int. Cl.: C07C 233/02, C07C 233/58, C07C 327/40, C07C 275/24, C07C 335/12, C07D 333/58, C07D 209/16, A61K 31/38, A61K 31/17, A61K 31/40

(54) **Nouveaux composés (hétéro)cycliques alkylés, leur procédé de préparation et les compositions pharmaceutiques qui les contiennent**
Alkyl(hetero)cyclische Derivate, Verfahren zu deren Herstellung und sie enthaltende pharmazeutische Zusammensetzungen
Alkyl(hetero)cyclic compounds, process for their preparation and pharmaceutical compositions containing them

(30) Priorité: 11.01.1995 FR 9500238
(43) Date de publication de la demande: 17.07.1996
(73) Titulaire: ADIR ET COMPAGNIE, 92415 Courbevoie Cédex (FR)
(72) Inventeur: Lesieur, Daniel, F-59147 Gondecourt (FR); Fourmaintraux, Eric, F-62200 St Martin/Boulogne s/Mer (FR); Depreux, Patrick, F-59280 Armentieres (FR); Delagrange, Philippe, F-92130 Issy-les-Moulineaux (FR); Renard, Pierre, F-78000 Versailles (FR); Guardiola-Lemaître, Béatrice, F-92210 Saint Cloud (FR)

(56) Documents cités:
- EP-A- 0 447 285
- EP-A- 0 527 687
- EP-A- 0 530 087
- EP-A- 0 562 956

## Description

L'invention concerne de nouveaux composés (hétéro)cycles alkylés, leur procédé de préparation et les compositions pharmaceutiques qui les contiennent.

On trouve dans l'art antérieur des composés (hétéro)cycliques en tant que ligands mélatoninergiques (EP 447 285, EP 527 687, EP 530 087, EP 562 956), mais aucun ne contient de groupement alkyle ou cycloalkyle comme substituant.

L'invention décrit de nouveaux composés (hétéro)cycliques alkylés qui s'avèrent être de puissants ligands des récepteurs mélatoninergiques.

De nombreuses études ont mis en évidence ces dix dernières années, le rôle capital de la mélatonine (5-méthoxy N-acétyl tryptamine) dans le contrôle du rythme circadien et des fonctions endocrines, et les récepteurs de la mélatonine ont été caractérisés et localisés.

Outre leur action bénéfique sur les troubles du rythme circadien (J. Neurosurg 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Secretions, 1990, 8 (3-4), pp 264-272) et analgésiques (Pharmacopsychiat., 1987, 20, pp 222-223) ainsi que pour le traitement de la maladie de Parkinson (J. Neurosurg 1985, 63, pp 321-341) et d'Alzheimer (Brain Research, 1990, 528, pp 170-174). De même, ces composés ont montré une activité sur certains cancers (Melatonin - clinical Perspectives, Oxford University Press, 1988, page 164-165), sur l'ovulation (Science 1987, 227, pp 714-720), et sur le diabète (Clinical endocrinolgy, 1986, 24, pp 359-364).

Des composés permettant d'agir sur le système mélatoninergique sont donc pour le clinicien d'excellents médicaments pour le traitement des pathologies mentionnées précédemment.

L'invention concerne les composés de formule (I): 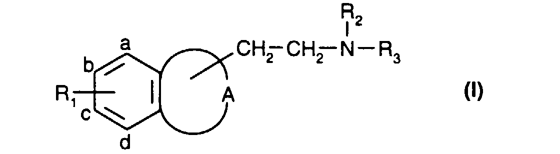 dans laquelle :
- R₁ représente un radical choisi parmi alkyle, alkyle substitué, cycloalkyle, cycloalkyle-substitué, cycloalkylalkyle, et cycloalkylalkyle substitué,
- A forme avec le noyau benzènique auquel il est lié un groupement cyclique choisi parmi tétrahydronaphtalène, dihydronaphtalène, naphtalène, benzothiophène, 2,3-dihydrobenzothiophène, indoline, indoline substitué, indole et indole substitué,
- R₂ représente un hydrogène ou un alkyle,
- R₃ représente :
   - un groupement R₃₁ : 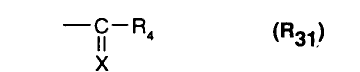 avec X représentant un soufre ou un oxygène et R₄ représentant un hydrogène ou un radical R₄₁ choisi parmi alkyle, alkyle substitué, alcényle, alcynyle, cycloalkyle, cycloalkyle substitué, cycloalkylalkyle et cycloalkylalkyle substitué,
   - ou un groupement de formule (R₃₂) : 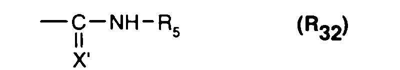 avec X' représentant un soufre ou un oxygène et R₅ représentant un hydrogène ou un radical choisi parmi alkyle, alkyle substitué, cycloalkyle, cycloalkyle substitué, cycloalkylalkyle et cycloalkylalkyle substitué,
   étant entendu que lors de la description de la formule (I), et sauf mention contraire :
   - les termes "alkyle" et "alkoxy" désignent des groupements linéaires ou ramifiés contenant de 1 à 6 atomes de carbone,
   - les termes "alcényle" et "alcynyle" désignent des groupements linéaires ou ramifiés contenant de 2 à 6 atomes,
   - le terme "cycloalkyle" désigne un groupement de 3 à 8 atomes de carbone,
   - le terme "substitué" associé au radical alkyle signifie que ce radical est substitué par un ou plusieurs substituants choisis parmi halogène, "alkyle", hydroxy et alkoxy,
   - le terme "substitué" associé au radical "cycloalkyle" et "cycloalkylalkyle" signifie que ce radical est substitué par un ou plusieurs radicaux ou groupements choisis parmi halogène, alkyle et oxo,
   - le terme "substitué" associé aux termes "indole" et "indoline" signifie que ces groupements sont substitués sur l'azote en position 1 par un radical choisi parmi -Ra, -CO-Ra et -CO-O-Ra dans lequel Ra représente un radical alkyle, phényle ou phénylalkyle,
   et leurs énantiomères et diastéréoisomères.

L'invention concerne particulièrement les composés de formule (I) dans laquelle, pris séparément ou ensemble,
- R₁ représente un alkyle,
- R₁ représente un (C₂-C₆) alkyle,
- R₁ représente un éthyle,
- R₁ représente un propyle,
- R₁ représente un butyle,
- A forme avec le noyau benzènique auquel il est lié un tétrahydronaphtalène,
- A forme avec le noyau benzènique auquel il est lié un naphtalène,
- A forme avec le noyau benzènique auquel il est lié un dihydronaphtalène,
- A forme avec le noyau benzènique auquel il est lié un benzothiophène,
- A forme avec le noyau benzénique auquel il est lié un indole,
- A forme avec le noyau benzénique auquel il est lié un indole substitué,
- R₂ représente un hydrogène,
- R₂ représente un alkyle,
- R₃ représente un groupement R₃₁ tel que défini dans la formule (I),
- R₃ représente un groupement R₃₂ tel que défini dans la formule (I),
- R₄ représente un atome d'hydrogène,
- R₄ représente un alkyle,
- R₄ représente un cycloalkyle,
- R₄ représente un alcényle,
- R₅ représente un hydrogène,
- R₅ représente un alkyle,
- R₅ représente un cycloalkyle,
- X représente un oxygène,
- X représente un soufre,
- X' représente un oxygène,
- ou X' représente un soufre.

Par exemple, l'invention concerne les composés particuliers de formule (I) répondant aux formules respectives (1) à (5) :

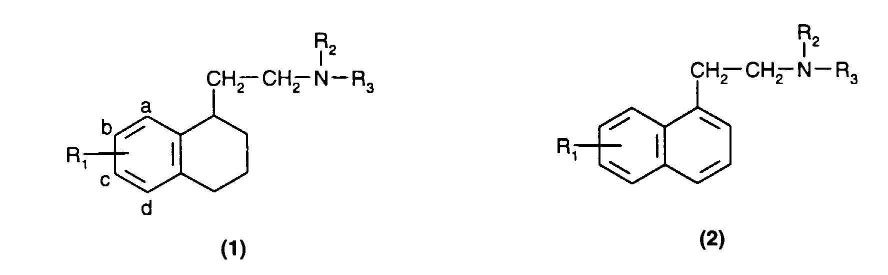

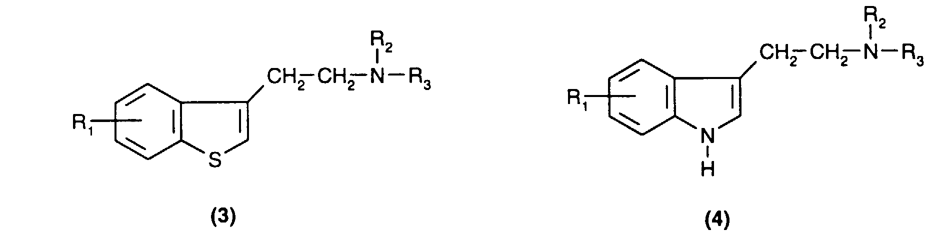

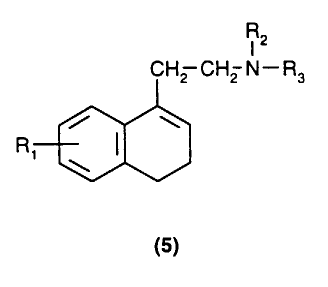

L'invention concerne particulièrement les composés de formule (I), par exemple les composés particuliers de formule (1) à (5), tels que définis ci-dessus dans laquelle R₁ est:
- en position a du noyau benzènique,
- en position b du noyau benzénique,
- en position c du noyau benzènique,
- ou en position d du noyau benzènique.

Par exemple, l'invention concerne les composés de formule (I) dans laquelle R₁ est en position b du noyau benzo.

De façon particulière, l'invention concerne les composés suivants :
- N-[2-(5-éthyl benzothiophèn-3-yl)éthyl]acétamide,
- N-[2-(5-éthyl benzothiophèn-3-yl)éthyl]cyclobutanecarboxamide,
- N-[2-(7-éthyl-1,2,3,4-tétrahydronapht-1-yl)éthyl]acétamide,
- N-[2-(7-éthyl-1,2,3,4-tétrahydronapht-1-yl)éthyl]butyramide,
- N-[2-(7-éthyl-1,2,3,4-tétrahydronapht-1-yl)éthyl]cyclopropanecarboxamide,
- N-[2-(7-éthyl-1,2,3,4-tétrahydronapht-1-yl)éthyl]pentanamide,
- et le N-[2-(7-éthyl-1,2,3,4-tétrahydronapht-1-yl)éthyl]trifluoroacétamide.

De façon particulière, les radicaux alkyles présents dans la formule (I) peuvent être choisis parmi méthyle, éthyle, n-propyle, isopropyle, n-butyle, isobutyle, sec-butyle, tert-butyle, pentyle, ou hexyle.

Les radicaux alkoxy présents dans la formule (I) peuvent être choisis parmi méthoxy, éthoxy, n-propoxy, isopropoxy, n-butoxy, iso-butoxy, sec-butoxy, tert-butoxy, pentyloxy, et hexyloxy.

Les halogènes présents dans la formule (I) peuvent être choisis parmi le brome, le chlore, le fluor, et l'iode.

Les cycloalkyles présents dans la formule (I) peuvent être choisis parmi cyclopropyle, cyclobutyle, cyclopentyle, cyclohexyle, cycloheptyle et cyclooctyle.

L'invention concerne également le procédé de préparation des composés de formule (I) caractérisé en ce que :
on fait réagir un composé de formule (II) : 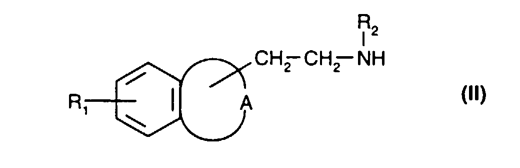 dans laquelle R₁ et A sont tels que définis dans la formule (I),
- soit avec l'acide formique ou avec un composé de formule (IIIa) ou (IIIb) : 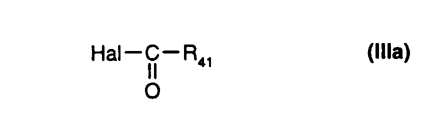 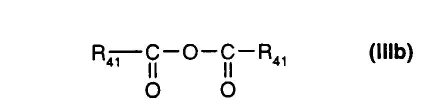 dans laquelle R₄₁ est tel que défini dans la formule (I) et Hal représente un halogène, afin d'obtenir les composés de formule (I/a): 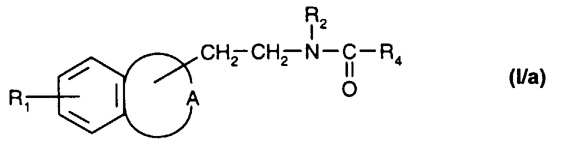 dans laquelle R₁, R₂, R₄ et A sont tels que définis précédemment,
   composés de formule (I/a) soumis au réactif de Lawesson pour obtenir les composés de formule (I/a'): 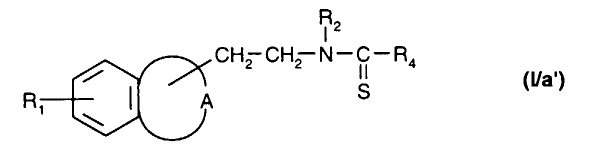 dans laquelle R₁, R₂, R₄ et A sont tels que définis précédemment,
- soit avec un composé de formule (IV) :

   X'=C=N-R₅ (IV)

   dans laquelle X' et R₅ sont tels que définis dans la formule (I) afin d'obtenir les composés de formule (I/b) : 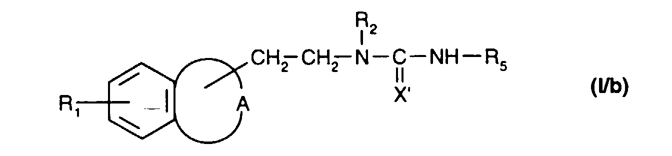 dans laquelle R₁, R₂, R₅, A et X' sont tels que définis précédemment, les composés de formule (I/a), (I/a') et (I/b) formant l'ensemble des composés de formule (I), composés de formule (I) qui sont, le cas échéant, séparés en leurs différents énantiomères ou diastéréoisomères.

Par exemple, l'invention s'étend au procédé de préparation des composés de formule (I/c): 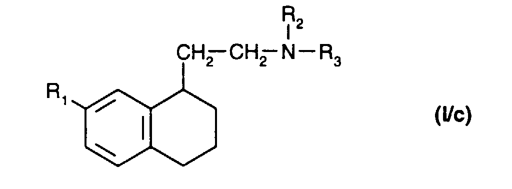 dans laquelle R₁, R₂ et R₃ sont tels que définis dans la formule (I),
caractérisé en ce que :
on fait réagir un composé de formule (Il/a) : 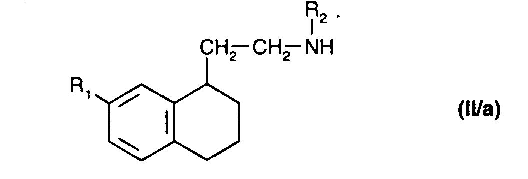 dans laquelle R₁ et R₂ sont tels que définis précédemment,
- soit avec un composé de formule (IIIa) ou (IIIb) tels que définis précédemment, afin d'obtenir les composés de formule (I/d) : 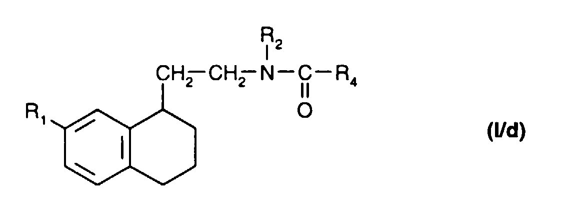 dans laquelle R₁, R₂ et R₄ sont tels que définis précédemment, qui sont ensuite soumis au réactif de Lawesson pour obtenir les composés de formule (I/d') : 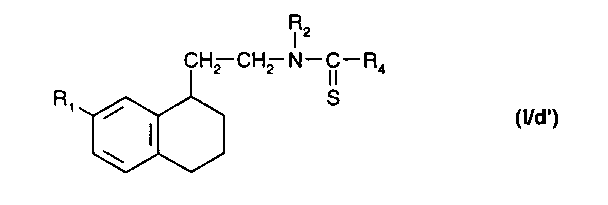 dans laquelle R₁, R₂, et R₄ sont tels que définis précédemment,
- soit avec un composé de formule (IV) tel que défini précédemment, afin d'obtenir les composés de formule (I/e) : 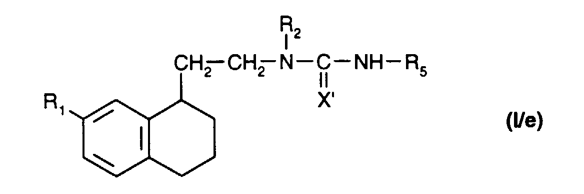 dans laquelle R₁, R₂, R₅ et X' sont tels que définis précédemment,
   les composés de formule (I/d), (I/d') et (I/e) formant l'ensemble des composés de formule (I/c),
   les composés de formule (I/c) pouvant être séparés en leurs différents énantiomères ou diastéréoisomères.

Par exemple, l'invention s'étend également au procédé de préparation des composés de formule (I/f): 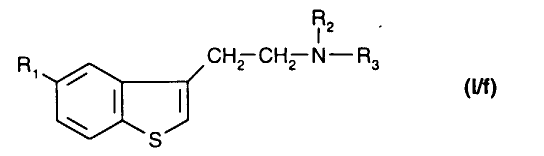 dans laquelle R₁, R₂ et R₃ sont tels que définis dans la formule (I),
caractérisé en ce que :
on fait réagir un composé de formule (Il/b) : 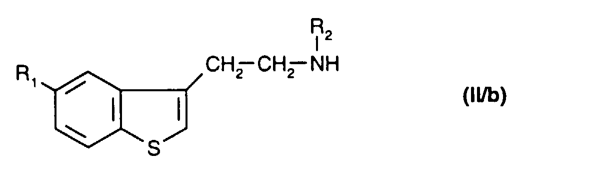 dans laquelle R₁ et R₂ sont tels que définis précédemment,
- soit avec un composé de formule (IIIa) ou (IIIb) tels que définis précédemment, afin d'obtenir les composés de formule (I/g): 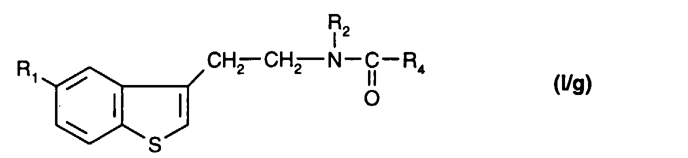 dans laquelle R₁, R₂ et R₄ sont tels que définis précédemment, qui sont ensuite soumis au réactif de Lawesson pour obtenir les composés de formule (I/g'): 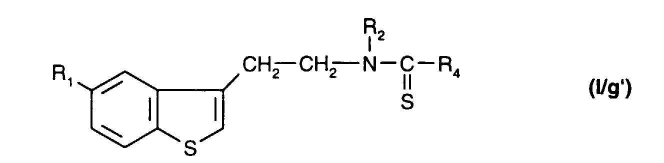 dans laquelle R₁, R₂ et R₄ sont tels que définis précédemment,
- soit avec un composé de formule (IV) tel que défini précédemment, afin d'obtenir les composés de formule (I/h): 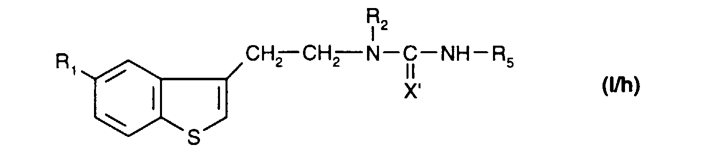 dans laquelle R₁, R₂, R₅ et X' sont tels que définis précédemment,
   les composés de formule (I/g), (I/g') et (I/h) formant l'ensemble des composés de formule (I/f),
   les composés de formule (I/f) pouvant être séparés en leurs différents énantiomères ou diastéréoisomères.

Les matières premières utilisées dans les procédés précédemment décrits sont soit commerciales, soit aisément accessibles à l'homme du métier d'après la littérature et les exemples de préparations données ci-après.

Par exemple, il est possible de préparer les composés de formule (Il/a) : 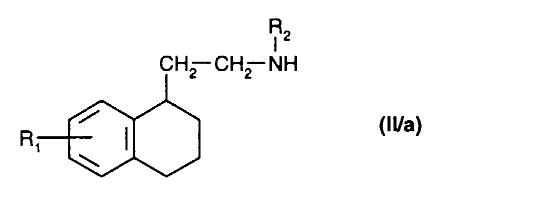 dans laquelle R₁ et R₂ sont tels que définis dans la formule (I), par réaction d'un composé de formule (V) : 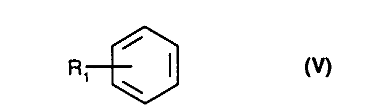 dans laquelle R₁ est tel que défini précédemment avec l'anhydride succinique pour obtenir un composé de formule (VI) : 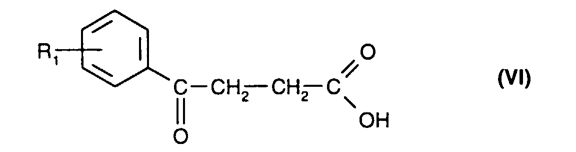 dans laquelle R₁ est tel que défini précédemment,
qui est réduit pour obtenir un composé de formule (VII) : 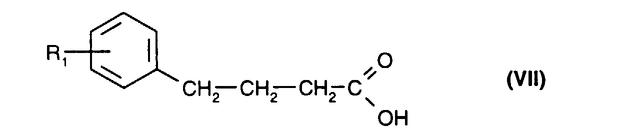 dans laquelle R₁ est tel que défini précédemment,
qui est ensuite cyclisé pour obtenir un composé de formule (VIII) : 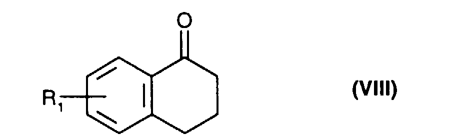 dans laquelle R₁ est tel que défini précédemment,
qui est mis en réaction avec du diéthylique cyanométhyl phosphonate pour obtenir le composé de formule (IX): 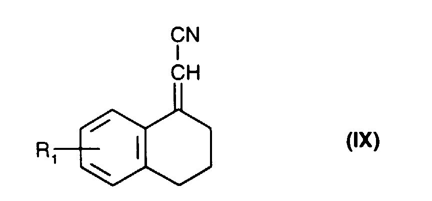 dans laquelle R₁ est tel que défini précédemment, qui est ensuite hydrogéné pour obtenir le composé de formule (Il/c) : 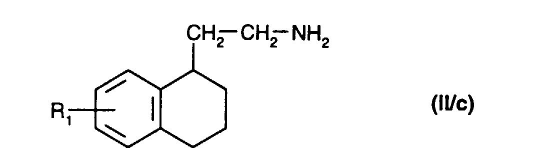 dans laquelle R₁ est tel que défini précédemment, composé de formule (Il/c) éventuellement alkylé sur la fonction amine pour obtenir un
composé de formule (Il/d) : 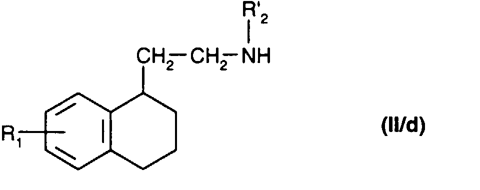 dans laquelle R₁ est tel que défini précédemment et R'₂ représente un radical (C₁-C₆)alkyle,
les composés de formule (Il/c) et (Il/d) formant l'ensemble des composés de formule (Il/a),
les composés de formule (Il/a) pouvant être séparés en leurs énantiomères ou diastéréoisomères et salifiés par un acide pharmaceutiquement acceptable.

L'aromatisation des composés à structure tétrahydronaphtalénique tels que décrits ci-dessus permet d'obtenir les composés utiles pour la préparation des composés de formule (I) dans lesquels A forme avec le noyau benzène auquel il est lié un groupement naphtalène.

Un autre exemple de préparation des composés de formule (Il) consiste dans le procédé de préparation des composés de formule (Il/e) : 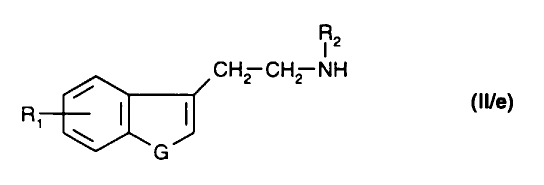 dans laquelle R₁ et R₂ sont tels que définis dans la formule (I) et G représente un soufre ou un groupement -NH, caractérisé en ce que on fait réagir un composé de formule (X) : 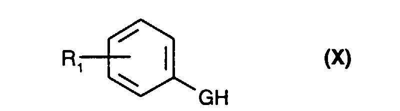 dans laquelle R₁ et G sont tels que définis précédemment, avec le 4-chloroacétoacétate d'éthyle afin d'obtenir le composé de formule (XI) : 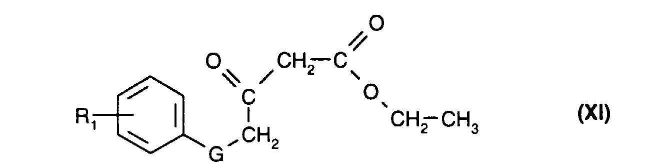 dans laquelle R₁ et G sont tels que définis précédemment, que l'on cyclise, pour obtenir un composé de formule (XII), 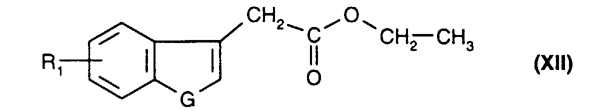 dans laquelle R₁ et G sont tels que définis précédemment, que l'on hydrolyse pour obtenir le composé de formule (XIII) : 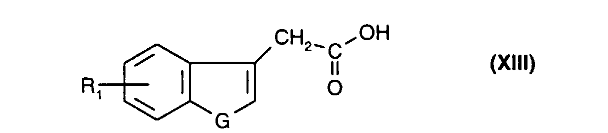 dans laquelle R₁ et G sont tels que définis précédemment, que l'on amidifie pour obtenir un composé de formule (XIV) : 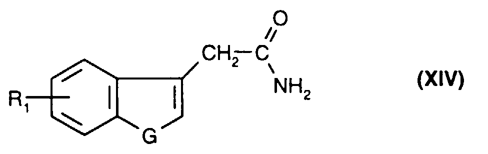 dans laquelle R₁ et G sont tels que définis précédemment, que l'on déshydrate en nitrile puis qui est ensuite réduit pour obtenir un composé de formule (Il/f) : 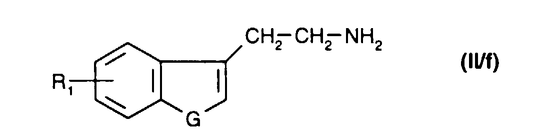 dans laquelle R₁ et G sont tels que définis précédemment, composé de formule (Il/e) éventuellement alkylé sur la fonction amine pour obtenir un composé de formule (Il/g) : 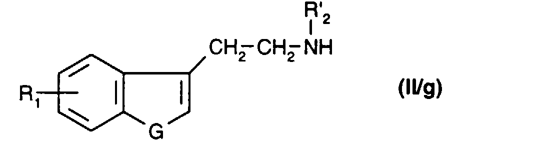 dans laquelle R₁ et G sont tels que définis précédemment et R'₂ représente un radical (C₁-C₆) alkyle,
les composés de formule (II/f) et (II/g) formant l'ensemble des composés de formule (Il/d), les composés de formule (Il/e) pouvant être salifiés par un acide pharmaceutiquement acceptable.

Plus particulièrement, la préparation des composés de formule (Il/d) est accessible quand G représente un soufre.

Parmi les acides pharmaceutiquement acceptables que l'on peut utiliser pour former un sel d'addition avec les composés de formule (II), on peut citer à titre d'exemples et de façon non limitative, les acides chlorhydrique, sulfurique, phosphorique, tartrique, malique, maléique, fumarique, oxalique, méthanesulfonique, éthanesulfonique, camphorique, et citrique.

Les composés de formule (I) possèdent des propriétés pharmacologiques très intéressantes pour le clinicien.

Les composés de l'invention et les compositions pharmaceutiques les contenant s'avèrent être utiles pour le traitement des troubles du système mélatoninergique.

L'étude pharmacologique des composés de l'invention a en effet montré qu'ils n'étaient pas toxiques, doués d'une très haute affinité sélective pour les récepteurs de la mélatonine et possédaient d'importantes activités sur le système nerveux central et en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, de la maladie de Parkinson, de la démence sénile, des divers désordres liés au vieillissement normal ou pathologique, de la migraine, des pertes de mémoire, de la maladie d'Alzheimer, ainsi que les troubles de la circulation cérébrale. Dans un autre domaine d'activité, il apparaît que les produits de l'invention possèdent des propriétés d'inhibiteurs de l'ovulation, d'immunomodulateurs et qu'ils sont susceptibles d'être utilisés dans le traitement anticancéreux.

Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.

Par exemple, les composés seront utilisés dans le traitement des dépressions saisonnières et des troubles du sommeil.

La présente invention a également pour objet les compositions pharmaceutiques contenant les produits de formule (I) en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,1 mg et 1 g par 24 heures en 1 ou 2 prises, plus particulièrement entre 1 à 100 mg, par exemple entre 1 à 10 mg.

Les exemples suivants illustrent l'invention, mais ne la limitent en aucune façon.

### PREPARATION 1 : 2-(7-ETHYL-1,2,3-4-TETRAHYDRONAPHT-1-YL)ETHYLAMINE

### STADE A: ACIDE 4-OXO-4-(4-ETHYL-PHENYL)BUTYRIQUE

| **Réactifs :** | |
|---|---|
| Ethyl benzène | 0,05 mole (5 cm³) |
| Chlorure d'aluminium | 0,02 mole (2,6 g) |
| Anhydride succinique | 0,01 mole (1 g) |

### Mode opératoire :

Dans une fiole de 50 cm³, mélanger sous agitation magnétique 5 cm³ d'éthyl benzène et 2,6 g de chlorure d'aluminium. Refroidir la solution dans un bain de glace puis ajouter 1 g d'anhydride succinique. Agiter 1 h 30 à la température de 0°C puis 3 h à température ambiante.
Verser le mélange réactionnel dans la glace.
Acidifier par addition d'acide chlorhydrique 1N (pH 3-4). Extraire par 3 volumes d'éther. Les phases organiques sont lavées 3 fois par une solution de carbonate de potassium à 10 %. Les phases aqueuses sont rassemblées et acidifiées par addition d'acide chlorhydrique concentré.
Le précipité obtenu est essoré puis recristallisé.

### Caractéristiques :

206,23 g/mole pour C₁₂H₁₄O₃
Poudre blanche
Température de fusion : 106-108°C
Rf = 0,36 éluant: acétone-toluène-cyclohexane (2-2-1)
Solvant de recristallisation : cyclohexane
Rendement : 57 %

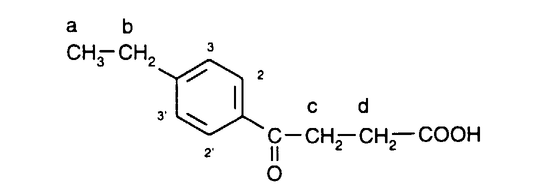

| **Analyse spectroscopique dans l'infrarouge :** | |
|---|---|
| 2960-2920 cm⁻¹ | υ CH alkyles |
| 1710 cm⁻¹ | υ CO acide |
| 1670 cm⁻¹ | υ CO cétone |
| 1600 cm⁻¹ | υ C=C aromatiques |

| **Analyse spectroscopique de RMN du proton (80 MHz, DMSO d**_{**6**}**, δ) :** | | |
|---|---|---|
| 1,2 ppm (triplet, 3H) | CH₃ (a) | J_{a-b} = 6,60 Hz |
| 2,6 ppm (multiplet, 4H) | CH₂ (b) et CH₂ (d) | Jb-a = Jd-c = 6,60 Hz |
| 3,2 ppm (triplet, 2H) | CH₂ (c) | Jc-d = 6,60 Hz |
| 7,4 ppm (doublet, 2H) | H₃ et H₅ | Jₒᵣₜₕₒ = 8,80 Hz |
| 7,9 ppm (doublet, 2H) | H₂ et H₆ | Jₒᵣₜₕₒ = 8,80 Hz |
| 12,1 ppm (multiplet, 1H) | COOH | |

| **Analyse spectrométrique de masse** | | |
|---|---|---|
| m/e | 206 | M⁺ |
| m/e | 207 | (M + 1)⁺ |

### STADE B : ACIDE 4-(4-ETHYL-PHENYL)BUTYRIQUE

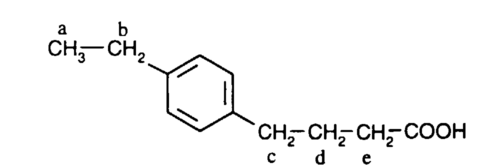

| **Réactifs :** | |
|---|---|
| Acide-4-oxo-4-(4-éthyl-phényl) butyrique (stade A) | 0,012 mole (2,5 g) |
| Triéthylsilane | 0,028 mole (3,2 g) |
| Acide trifluoroacétique | 0,12 mole (19 cm³) |

### Mode opératoire :

Dans une fiole de 100 cm³, 2,5 g d'acide-4-oxo-4-(4-éthyl-phényl) butyrique sont dissous sous agitation magnétique dans 19 cm³ d'acide trifluoroacétique. Ajouter goutte à goutte 3,2 g de triéthylsilane.
Agiter 86 heures à température ambiante.
Le mélange réactionnel est versé dans la glace.
Extraire par 3 volumes d'éther. Les phases organiques sont lavées 3 fois par une solution de carbonate de potassium à 10 %. Les phases aqueuses sont rassemblées puis acidifiées par addition d'acide chlorhydrique concentré, jusqu'à pH 3-4.
Le précipité obtenu est essoré puis recristallisé.

### Caractéristiques :

192,25 g/mole pour C₁₂H₁₆O₂
Poudre blanche
Température de fusion : 71-73°C
Rf = 0,67, éluant: acétone-toluène-cyclohexane (2-2-1)
Solvant de recristallisation : eau
Rendement : 65 %

| **Analyse spectroscopique dans l'infrarouge** | |
|---|---|
| 3280-2780 cm⁻¹ | υ OH acide |
| 2940-2850 cm⁻¹ | υ CH alkyles |
| 1680 cm⁻¹ | υ CO acide |
| 1510 cm⁻¹ | υ C=C aromatiques |

| **Analyse spectroscopique de RMN du proton (300 MHz, DMSO d**_{**6**}**, δ) :** | | |
|---|---|---|
| 1,14 ppm (triplet, 3H) | CH₃ (a) | J_{a-b} = 7,63 Hz |
| 1,76 ppm (multiplet, 2H) | CH₂ (d) | |
| 2,20 ppm (triplet, 2H) | CH₂ (e) | J_{d-e} = 7,65 Hz |
| 2,55 ppm (multiplet, 4H) | CH₂ (c) et CH₂ (b) | |
| 7,11 ppm (multiplet, 6H) | H aromatiques | |
| OH acide non observé | | |

| **Analyse spectrométrique de masse :** | |
|---|---|
| m/e 192 | M⁺ |
| m/e 193 | (M + 1)⁺ |

### STADE C : 7-ETHYL TETRAL-1-ONE

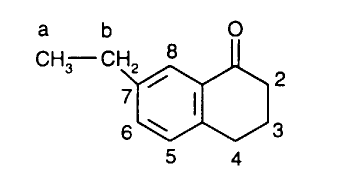

| **Réactifs :** | |
|---|---|
| Acide-4-(4-éthyl phényl) butyrique (stade B) | 0,013 mole (2,5 g) |
| Acide polyphosphorique | 25 g |

### Mode opératoire :

Dans un ballon à col rodé de 100 cm³, verser 25 g d'acide polyphosphorique. Ajouter 2,5 g d'acide 4-(4-éthyl phényl) butyrique. Agiter 6 h à une température de 45°C. Le mélange réactionnel est versé dans la glace. Extraire par 3 volumes d'éther. Les phases organiques sont lavées 3 fois par une solution de carbonate de potassium à 10 %, séchées sur sulfate de magnésium puis évaporées à sec.

L'huile obtenue est purifiée par chromatographie sur colonne.

### Caractéristiques :

174,23 g/mole pour C₁₂H₁₄O
Huile incolore
Rf = 0,35 éluant : toluène-cyclohexane (1-2)
Rendement : 55 %

| **Analyse spectroscopique dans l'infrarouge :** | |
|---|---|
| 3010 cm⁻¹ | ν CH aromatiques |
| 2980-2860 cm⁻¹ | ν CH alkyles |
| 1680 cm⁻¹ | ν CO cétone |
| 1605 cm⁻¹ | ν C=C aromatiques |

| **Analyse spectroscopique de RMN du proton (300 MHz, DMSO d**_{**6**}**, δ) :** | |
|---|---|
| 1,13 ppm (triplet, 3H) | CH₃ (a), J_{a-b} = 7,68 Hz |
| 2,01 ppm (multiplet, 2H) | CH₂ (3) |
| 2,59 ppm (multiplet, 4H) | CH₂ (b) et CH₂ (4) |
| 2,88 ppm (triplet, 2H) | CH₂ (2), J₂₋₃ = 5,77 Hz |
| 7,25 ppm (doublet, 1H) | H₅, Jₒᵣₜₕₒ = 8,59 Hz |
| 7,39 ppm (doublet dédoublé, 1H) | H₆, Jₒᵣₜₕₒ = 8,59 Hz, J_{méta} = 2,14 Hz |
| 7,70 ppm (doublet, 1H) | H₈, J_{méta} = 2,14 Hz |

| **Analyse spectrométrique de masse :** | |
|---|---|
| m/e 174 | M⁺ |
| m/e 175 | (M+1)⁺ |

### STADE D : 2-(7-ETHYL-1,2,3,4-TETRAHYDRONAPHT-YLIDEN-1-YL)ACETONITRILE

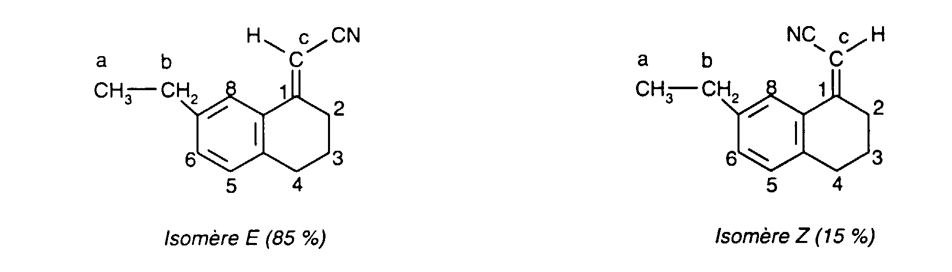

| **Réactifs :** | |
|---|---|
| 7-Ethyl tétralone (stade C) | 0,029 mole (5 g) |
| Cyanométhyl phosphonate diéthylique | 0,048 mole (8,9 g) |
| Hydrure de sodium | 0,048 mole (1,12 g) |
| Tétrahydrofurane anhydre | 20 cm³ |

### Mode opératoire :

Dans un ballon tricol de 100 cm³, faire barboter de l'azote gazeux dans 20 cm³ de tétrahydrofurane anhydre; 1,15 g d'hydrure de sodium sont additionnés sous agitation magnétique puis le cyanométhyl phosphonate diéthylique, goutte à goutte.

Le milieu réactionnel est agité 1 h à température ambiante, jusqu'à la fin du dégagement gazeux.
Ajouter la 7-éthyl tétralone puis continuer l'agitation 24 h à température ambiante, sous courant d'azote gazeux.
Verser le mélange réactionnel dans la glace.
Extraire par 3 volumes d'éther. Les phases organiques sont lavées 3 fois à l'eau, séchées sur sulfate de magnésium puis évaporées à sec.
L'huile obtenue est purifiée par chromatographie sur colonne.

### Caractéristiques :

197,27 g/mole pour C₁₄H₁₅N
Huile incolore
Rf = 0,60, éluant: acétone-toluène-cyclohexane (5-3-2)
Rendement : 60 %

| **Analyse spectroscopique dans l'infrarouge :** | |
|---|---|
| 3050 cm⁻¹ | ν CH aromatiques |
| 2960-2820 cm⁻¹ | ν CH alkyles |
| 2200 cm⁻¹ | ν CN |
| 1585 cm⁻¹ | ν C=C aromatiques |

| **Analyse spectroscopique de RMN du proton (300 MHz, DMSO d**_{**6**}**, δ) :** | |
|---|---|
| *Isomère E:* | |
| 1,22 ppm (triplet, 3H) | CH₃ (a), J_{a-b} = 8,04 Hz |
| 1,94 ppm (multiplet, 2H) | CH₂ (3) |
| 2,66 ppm (multiplet, 2H) | CH₂ (2) |
| 2,87 ppm (multiplet, 4H) | CH₂ (b) et CH₂ (4) |
| 5,73 ppm (singulet, 1H) | CH (c) |
| 7,08 ppm (doublet, 1H) | H₅, Jₒᵣₜₕₒ = 7,76 Hz |
| 7,16 ppm (multiplet, 1H) | H₆, Jₒᵣₜₕₒ = 7,76 Hz |
| 7,36 ppm (multiplet, 1H) | H₈ |

| *Isomère* Z: | |
|---|---|
| 1,22 ppm (triplet, 3H) | CH₃ (a), J_{a-b} = 8,04 Hz |
| 1,94 ppm (multiplet, 2H) | CH₂ (3) |
| 2,66 ppm (multiplet, 2H) | CH₂ (2) |
| 2,87 ppm (multiplet, 4H) | CH₂ (b) et CH₂ (4) |
| 5,23 ppm (singulet, 1H) | CH (c) |
| 7,08 ppm (doublet, 1H) | H₅, Jₒᵣₜₕₒ = 7,76 Hz |
| 7,16 ppm (multiplet, 1H) | H₆, Jₒᵣₜₕₒ = 7,76 Hz |
| 8,14 ppm (multiplet, 1H) | H₈ |

### STADE E: (R,S) 2-(7-ETHYL-1,2,3,4-TETRAHYDRO-NAPHT-1-YL)ETHYLAMINE

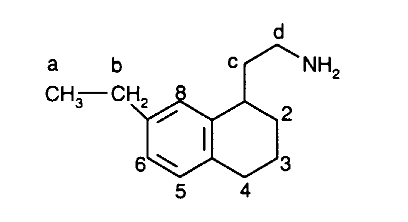

| **Réactifs :** | |
|---|---|
| 2-(7-Ethyl-1,2,3,4-tétrahydronapht-ylidèn-1-yl)acétonitrile (stade D) | 0,015 mole (3 g) |
| Alcool absolu | 150 cm³ |
| Nickel de Raney | 0,5 g |
| Hydrogène | 60 Bars |

### Mode opératoire :

Dans un autoclave de 250 cm³, dissoudre 3 g de 2-(7-éthyl-1,2,3,4-tétrahydronaphtylidèn-1-yl) acétonitrile dans 150 cm³ d'alcool absolu. Ajouter 0,5 g de Nickel de Raney. Agiter 6h à 60°C sous une pression d'hydrogène de 60 Bars.
Filtrer sous vide. Le filtrat est évaporé à sec.
Le résidu est repris par un petit volume d'éther saturé en acide chlorhydrique gazeux. Le précipité qui se forme est essoré puis recristallisé.

### Caractéristiques (chlorhydrate) :

239,78 g/mole pour C₁₄H₂₂ClN
Poudre blanche
Température de fusion : 116-118°C
Rf = 0,73, dans acétone-toluène-cyclohexane-triéthylamine (5-3-2-1)
Rendement : 49 %
Solvant de recristallisation : acétate d'éthyle

| **Analyse spectroscopique dans l'infrarouge :** | |
|---|---|
| 3250-2500 cm⁻¹ | ν NH₃⁺ |
| Disparition de la bande CN | |
| 1605 cm⁻¹ | ν C=C (aromatiques) |

| **Analyse spectroscopique de RMN du proton (300 MHz, DMSO d**_{**6**}**, δ) :** | | |
|---|---|---|
| 1,14 ppm (triplet, 3H) | CH₃ (a) | J_{a-b} = 7,04 Hz |
| 1,67 ppm (multiplet, 6H) | CH₂ (c), CH₂ (2) et CH₂ (3) | |
| 2,63 ppm (multiplet, 7H) | CH₂ (b), CH (d), CH (1) et CH₂ (4) | |
| 6,81-7,11 ppm (multiplet, 3H) | H aromatiques | |
| 8,00 ppm (multiplet, 3H) | NH₃⁺ | |

| **Analyse élémentaire :** | | | | |
|---|---|---|---|---|
| Calculé | C:70,12 % | H:9,25 % | N:5,83 % | Cl:14,79% |
| Trouvé | C:70,40 % | H:8,96 % | N:5,83 % | Cl:14,69% |

### PREPARATION 2: 2-(5-ETHYL-BENZO[b]THIOPHEN-3-YL)ETHYLAMINE

### STADE A :4-ETHYL BENZENE THIOL

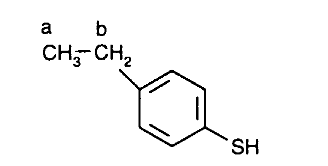

| **Réactifs :** | |
|---|---|
| Chlorure de l'acide 4-éthyl-benzène-sulfonique | 0,024 mole (5g) |
| Hydrure mixte d'aluminium et de lithium | 0,096 mole (3,6 g) |
| Tétrahydrofurane anhydre | 20 cm³ |

### Mode opératoire :

Dans un ballon de 100 cm³, ajouter sous agitation magnétique 3,6 g d'hydrure mixte d'aluminium et de lithium à 20 cm³ de tétrahydrofurane anhydre. Refroidir dans un bain de glace puis ajouter goutte à goutte 5 g du chlorure de l'acide 4-éthyl benzène sulfonique. Agiter pendant 3 h.
Verser le mélange réactionnel dans de la glace. Extraire la phase aqueuse par trois volumes d'éther. Les phases organiques sont lavées à l'eau, séchées sur sulfate de magnésium puis évaporées à sec.
L'huile obtenue est chromatographiée sur colonne.

### Caractéristiques :

138,23 g/mole pour C₈H₁₀S
Huile incolore
Rf = 0,85 dans acétone-toluène-cyclohexane (2-2-1)
Rendement: 70 %

| **Analyse spectroscopique dans l'infrarouge :** | |
|---|---|
| 3080 cm⁻¹ | υ CH aromatiques |
| 2960-2860 cm⁻¹ | υ CH alkyles |
| 2560 cm⁻¹ | υ SH |
| 1490 cm⁻¹ | υ C=C aromatiques |

| **Analyse spectroscopique de RMN du proton (80 MH**_{**Z**}**, DMSO d**_{**6**}**, δ) :** | |
|---|---|
| 1,25 ppm (triplet, 3H) | CH₃ (a), J_{a-b} = 7,80 Hz |
| 2,65 ppm (quintuplet, 2H) | CH₂ (b), J_{b-a} = 7,80 Hz |
| 3,40 ppm (singulet, 1H) | SH (atténué dans D₂O) |
| 7,20 ppm (multiplet, 4H) | H aromatiques |

### STADE B : 4-ETHYL-PHENYL-THIOACETOACETATE D'ETHYLE

| **Réactifs :** | |
|---|---|
| 4-éthyl-benzène-thiol (Stade A) | 0,025 mole (3,2 g) |
| 4-Chloro-acétoacétate d'éthyle | 0,026 mole (4,2 g) |
| Pyridine | 0,1 mole (8 cm³) |
| Ether anhydre | 10 cm³ |

### Mode opératoire :

Dans un ballon de 100 cm³, sous agitation magnétique, 3,2 g de 4-éthyl-benzènethiol et 8 g de pyridine sont dissous dans 10 cm³ d'éther anhydre. 4,2 g de 4-chloro acétoacétate d'éthyle sont ajoutés goutte à goutte.
La solution est agitée 2 h à température ambiante puis versée dans la glace.
La phase organique est extraite, lavée à l'eau, séchée sur sulfate de magnésium puis évaporée à sec.
L'huile obtenue est purifiée par chromatographie sur colonne.

### Caractéristiques :

266,22 g/mole pour C₁₄H₁₈O₃S
Huile incolore
Rf = 0,56 dans éther-hexane-éther de pétrole (2-2-1)
Rendement : 50 %

| **Analyse spectroscopique dans l'infrarouge :** | |
|---|---|
| 2960-2860 cm⁻¹ | υ CH alkyles |
| Disparition de la bande SH | |
| 1740 cm⁻¹ | υ CO ester |
| 1710 cm⁻¹ | υ CO cétone |
| 1490 cm⁻¹ | υ C=C aromatiques |

| **Analyse spectroscopique de RMN du proton (80 MHz, DMSO d**_{**6**}**, δ) :** | |
|---|---|
| 1,20 ppm (multiplet, 6H) | CH₃ (a) et CH3 (f) |
| 2,65 ppm (quintuplet, 2H) | CH₂ (b), J_{b-a} = 7,90 Hz |
| 3,70 ppm (singulet, 2H) | CH₂ (c) |
| 3,75 ppm (singulet, 2H) | CH₂ (d) |
| 4,20 ppm (quintuplet, 2H) | CH₂ (e), J_{e-f} = 7,90 Hz |
| 7,20 ppm (multiplet, 4H) | H aromatiques |

### STADE C : (5-ETHYL-BENZO[b]THIOPHEN-3-YL)ACETATE D'ETHYLE

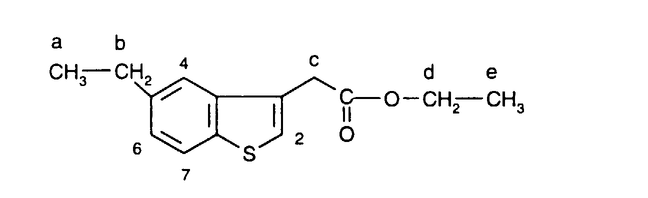

| **Réactifs :** | |
|---|---|
| 4-Ethyl-phényl-thioacétoacétate d'éthyle (stade B) | 0,012 mole (3 g) |
| Acide polyphosphorique | 30 g |
| Toluène | 25 cm³ |
| Pentoxyde de phosphore | 0,7 g |

### Mode opératoire :

Dans un ballon de 250 cm³ contenant 30 g d'acide polyphosphorique, ajouter 25 cm³ de toluène puis 0,7 g de pentoxyde de phosphore. Le 4-éthyl-phénylthioacétoacétate d'éthyle est ensuite additionné en une seule fois et le mélange réactionnel agité pendant 5 h à la température de 50°C.
Le milieu réactionnel est versé dans la glace. La phase aqueuse est extraite par 3 volumes d'éther. Les phases organiques sont rassemblées, lavées par 3 volumes d'eau, séchées sur sulfate de magnésium puis évaporées à sec.
L'huile obtenue est purifiée par chromatograhie sur colonne.

### Caractéristiques :

248,33 g/mole pour C₁₄H₁₆O₂S
Huile incolore
Rf = 0,74 éluant: éther-hexane-éther de pétrole (2-2-1)
Rendement : 55 %

| **Analyse spectroscopique dans l'infrarouge :** | |
|---|---|
| 2950-2860 cm⁻¹ | υ CH alkyles |
| 1730 cm⁻¹ | υ CO ester |
| Disparition de la bande CO cétone | |
| 1580 cm⁻¹ | υ C=C aromatiques |

| **Analyse spectroscopique de RMN du proton (80 MHz, DMSO d**_{**6**}**, δ) :** | | |
|---|---|---|
| 1,20 ppm (multiplet, 6H) | CH₃ (a) et CH₃ (e) | |
| 2,75 ppm (quintuplet, 2H) | CH₂ (b), J_{b-a} | = 6,95 Hz |
| 3,90 ppm (singulet, 2H) | CH₂ (c) | |
| 4,15 ppm (quintuplet, 2H) | CH₂ (d), J_{d-e} | = 6,90 Hz |
| 7,20 ppm (doublet dédoublé, 1H) | H₆ , Jₒᵣₜₕₒ | = 8,35 Hz, J_{méta} = 1,40 Hz |
| 7,60 ppm (multiplet, 2H) | H₂ et H₄ | |
| 7,90 ppm (doublet, 1H) | H₇ , Jₒᵣₜₕₒ | = 8,35 Hz |

| **Analyse élémentaire :** | | | |
|---|---|---|---|
| Calculé | C : 67,70 % | H : 6,49 % | O:12,88 % |
| Trouvé | C:67,64 % | H:6,54 % | O:12,88% |

### STADE D : ACIDE 2-(5-ETHYL-BENZO[b]THIOPHEN-3-YL) ACETIQUE

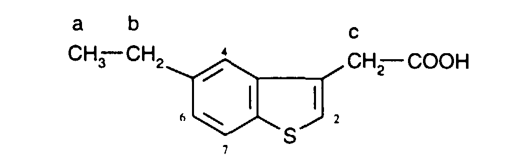

| **Réactifs :** | |
|---|---|
| (5-Ethyl-benzo[b]thiophèn-3-yl) acétate d'éthyle (stade C) | 0,012 mole (3 g) |
| Solution aqueuse de soude à 20 % | 5 cm³ |
| Méthanol | 10 cm³ |

### Mode opératoire :

Dans un ballon de 50 cm³, 3 g de 2-(5-éthyl-benzo[b]thiophène-3-yl) acétate d'éthyle sont dissous dans 10 cm³ de méthanol. Ajouter 5 cm³ d'une solution aqueuse de soude à 20 %. Le mélange réactionnel est agité à température ambiante pendant 14 h. Verser dans 50 cm³ d'eau puis extraire avec 2 volumes d'éther.
La phase aqueuse est acidifiée par addition d'une solution d'acide chlorhydrique concentrée (jusque pH 3-4).
Le précipité qui se forme est essoré puis recristallisé.

### Caractéristiques :

220,28 g/mole pour C₁₂H₁₂O₂S
Poudre blanche
Température de fusion : 125-127°C
Rf = 0,74, éluant : éther-hexane-éther de pétrole (2-2-1)
Solvant de recristallisation : alcool 95°-eau (1-7)
Rendement: 50 %

| **Analyse spectroscopique dans l'infrarouge :** | |
|---|---|
| 3200-2900 cm⁻¹ | υ OH acide |
| 2960-2840 cm⁻¹ | υ CH alkyles |
| 1705 cm⁻¹ | υ CO acide |

Disparition de la bande CO ester

| **Analyse spectroscopique de RMN du proton (300 MHz, DMSO d**_{**6**}**, δ) :** | |
|---|---|
| 1,18 ppm (triplet, 3H) | CH₃ (a) J_{a-b} = 7,58 Hz |
| 2,78 ppm (quintuplet, 2H) | CH₂ (b) J_{b-a} = 7,58 Hz |
| 3,89 ppm (singulet, 2H) | CH₂ (c) |
| 7,22 ppm (multiplet, 1H) | H₆ |
| 7,36 ppm (singulet, 1H) | H₂ |
| 7,56 ppm (multiplet, 1H) | H₄ |
| 7,76 ppm (multiplet, 1H) | H₇ Jₒᵣₜₕₒ = 8,33 Hz |
| 9,50-10,50 ppm (massif, 1H) | COOH |

| **Analyse élémentaire :** | | | | |
|---|---|---|---|---|
| Calculé | C : 65,42 % | H : 5,49 % | S : 14,56 % | O : 14,53 % |
| Trouvé | C : 65,32 % | H : 5,53 % | S : 14,65 % | O : 14,50 % |

### STADE E : (5-ETHYL-BENZO[b]THIOPHEN-3-YL)ACETAMIDE

| **Réactifs :** | |
|---|---|
| Acide 2-(5-éthyl-benzo[b]thiophèn-3-yl)acétique (Stade D) | 0,006 mole (1,4 g) |
| Chlorure de thionyle | 0,024 mole (2,9 g) |
| Chloroforme | 15 cm³ |
| Solution d'ammoniaque à 28 % | 25 cm³ |

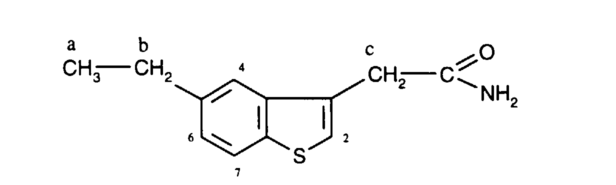

### Mode opératoire :

Dans une fiole de 100 cm³, 1,4 g d'acide 2-(5-éthyl benzo[b]thiophèn-3-yl) acétique sont dissous sous agitation magnétique dans 15 cm³ de chloroforme. 2,86 g de chlorure de thionyle sont ajoutés goutte à goutte.
La solution est agitée pendant 3 h à la température ambiante puis évaporée sous vide.
Le résidu est repris dans 30 cm³ d'éther puis filtré sur papier.
Le filtrat est refroidi dans un bain de glace.
25 cm³ d'une solution d'amoniaque à 28 % sont alors ajoutés en une seule fois.
Le précipité est essoré puis recristallisé.

### Caractéristiques :

219,29 g/mole pour C₁₂H₁₃NOS
Poudre blanche
Température de fusion : 201-203°C
Rf = 0,35, éluant : acétone-toluène-cyclohexane (5-3-2)
Solvant de recristallisation : hexane
Rendement: 65 %

| **Analyse spectroscopique dans l'infrarouge :** | |
|---|---|
| 3340 et 3160 cm⁻¹ | υ NH₂ amide |
| 2940-2840 cm⁻¹ | υ CH alkyles |
| Disparition de la bande CO acide | |
| 1650 cm⁻¹ | υ CO amide |

| **Analyse spectroscopique de RMN du proton (80 MHz, DMSO d**_{**6**}**, δ) :** | | |
|---|---|---|
| 1,25 ppm (triplet, 3H) | CH₃ (a) | J_{a-b} = 7,40 Hz |
| 2,75 ppm (quintuplet, 2H) | CH₂ (b) | J_{b-a} = 7,40 Hz |
| 3,60 ppm (singulet, 2H) | CH₂ (c) | |
| 7,00 ppm (multiplet, 2H) | NH₂ | |
| 7,20 ppm (doublet dédoublé, | 1H) ; H₆ | Jₒᵣₜₕₒ = 8,30 Hz J_{méta} = 1,40Hz |
| 7,50 ppm (singulet, 1H) | H₂ | |
| 7,65 ppm (doublet, 1H) | H₄ | J_{méta} = 1,40 Hz |
| 7,85 ppm (doublet, 1H) | H₇ | Jₒᵣₜₕₒ = 8,30 Hz |

| **Analyse élémentaire :** | | | |
|---|---|---|---|
| Calculé | C : 65,72 % | H : 5,97 % | N : 6,39 % |
| Trouvé | C : 65,91 % | H : 6,05 % | N : 6,59 % |

### STADE F : (5-ETHYL-BENZO[b]THIOPHEN-3-YL)ACETONITRILE

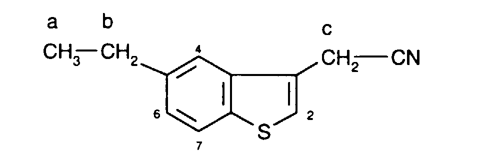

| **Réactifs :** | |
|---|---|
| (5-Ethyl benzo[b]thiophèn-3-yl)acétamide (Stade E) | 0,0011 mole (0,25 g) |
| Triéthylamine | 0,0025 mole (0,25 g) |
| Anhydride trifluoroacétique | 0,0012 mole (0,27 g) |
| Tétrahydrofurane anhydre | 5 cm³ |

### Mode opératoire :

Dans une fiole de 50 cm³, dissoudre sous agitation magnétique 0,25 g de 2-(5- éthyl-benzo[b]thiophèn-3-yl) acétamide dans 5 cm³ de tétrahydrofurane anhydre puis ajouter 0,25 g de triéthylamine.
Refroidir le mélange réactionnel dans un bain de glace-sel et ajouter goutte à goutte 0,27 g d'anhydride trifluoroacétique.
Agiter la solution pendant 1 h puis évaporer sous vide.
Le résidu est repris dans l'eau et le précipité essoré puis recristallisé.

### Caractéristiques :

201,28 g/mole pour C₁₂H₁₁NS
Poudre blanche
Point de fusion : 59-60°C
Rf = 0,82, éluant : acétone-toluène-cyclohexane (5-3-2)
Rendement : 62 %
Solvant de recristallisation : alcool 95 ° -eau (4-1)

| **Analyse spectroscopique dans l'infrarouge :** | |
|---|---|
| Disparition des bandes NH₂ d'amide | |
| 2940-2830 cm⁻¹ | υ CH alkyles |
| 2230 cm⁻¹ | υ CN |

Disparition de la bande CO d'amide

| **Analyse spectroscopique de RMN du proton (80 MHz, DMSO d**_{**6**}**, δ) :** | | |
|---|---|---|
| 1,25 ppm (triplet, 3H) | CH₃ (a) | J_{a-b} = 7,50 Hz |
| 2,80 ppm (quintuplet, 2H) | CH₂ (b) | J_{b-a} = 7,50 Hz |
| 4,25 ppm (singulet, 2H) | CH₂ (c) | |
| 7,30 ppm (doublet dédoublé, 1H) | H₆ | Jₒᵣₜₕₒ = 8,30 Hz |
| | | J_{méta} = 1,30 Hz |
| 7,70 ppm (multiplet, 2H) | H₂ et H₄ | |
| 7,95 ppm (doublet, 1H) | H₇ | Jₒᵣₜₕₒ = 8,30 Hz |

| **Analyse élémentaire :** | | | |
|---|---|---|---|
| Calculé | C : 71,60 % | H : 5,51 % | N : 6,96 % |
| Trouvé | C : 71,78 % | H : 5,68 % | N : 6,99 % |

### STADE G : 2-(5-ETHYL-BENZO[b]THIOPHEN-3-YL) ETHYLAMINE

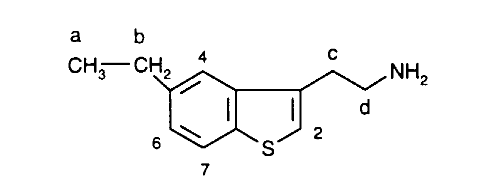

| **Réactifs :** | |
|---|---|
| (5-Ethyl-benzo[b]thiophèn-3-yl)acétonitrile (stade F) | 0,004 mole (0,7 g) |
| Hydrure de lithium et d'aluminium | 0,01 mole (0,4 g) |
| Chlorure d'aluminium | 0,01 mole (1,4 g) |
| Ether anhydre | 25 cm³ |

### Mode opératoire :

Dans une fiole de 100 cm³ contenant 25 cm³ d'éther anhydre, ajouter sous agitation magnétique 0,4 g d'hydrure de lithium et d'aluminium, 1,4 g de chlorure d'aluminium puis 0,7 g de (5-éthyl-benzo[b]thiophèn-3-yl)acétonitrile.
Au bout de 30 min, hydrolyser le mélange réactionnel sur de la glace et ajouter 20 cm³ d'une solution aqueuse de soude à 20 %.
La phase éthérée est extraite, lavée par 2 volumes d'eau, séchée sur sulfate de magnésium puis filtrée sur papier.
Faire barboter un courant d'acide chlorhydrique gazeux dans la solution et essorer le précipité formé
Le chlorhydrate est purifié par trituration dans le cyclohexane.

### Caractéristiques (chlorhydrate) :

241,77 g/mole pour C₁₂H₁₆CINS
Poudre blanche
Température de fusion : 159-161°C
Rf = 0,15, éluant : acétone-toluène-cyclohexane-triéthylamine (5-3-2-1)
Rendement : 50 %

| **Analyse spectroscopique dans l'infrarouge :** | |
|---|---|
| 3240-2600 cm⁻¹ | υ NH₃⁺ |
| Disparition de la bande CN | |

| **Analyse spectroscopique de RMN du proton (80 MHz, CDCl**_{**3**}**, δ) :** | | |
|---|---|---|
| 1,30 ppm (triplet, 3H) | CH₃ (a) | J_{a-b} = 7,50 Hz |
| 2,75 ppm (quintuplet, 2H) | CH₂ (b) | J_{b-a} = 7,50 Hz |
| 3,15 ppm (multiplet, 4H) | CH₂ (c) et CH₂ (d) | |
| 7,20 ppm (multiplet, 1H) | H₆ | Jₒᵣₜₕₒ = 8,35 Hz |
| 7,50 ppm (multiplet, 1H) | H₂ | |
| 7,70 ppm (multiplet, 1H) | H₄ | |
| 7,95 ppm (doublet, 1H) | H₇ | Jₒᵣₜₕₒ = 8,35 Hz |
| 8,20 ppm (multiplet, 3H) | NH₃⁺ | |

| **Analyse élémentaire :** | | | | |
|---|---|---|---|---|
| Calculé | C : 59,61 % | H : 6,67% | N : 5,79 % | Cl : 14,66 % |
| Trouvé | C : 59,78 % | H : 6,78% | N : 5,47 % | Cl : 14,28 % |

### PREPARATION 3 : (R,S) 2-(7-METHYL-1,2,3,4-TETRAHYDRO-NAPHT-1-YL) ETHYLAMINE

En procédant comme dans la préparation 1 mais en partant du toluène au lieu de l'éthylbenzène, on obtient le composé du titre.

### PREPARATION 4 : (R,S) 2-(7-PROPYL-1,2,3,4-TETRAHYDRO-NAPHT-1-YL) ETHYLAMINE

En procédant comme dans la préparation 1, mais en remplaçant l'éthylbenzène par le propylbenzène, on obtient le composé du titre.

### PREPARATION 5 : (R,S) 2-(7-BUTYL-1,2,3,4-TETRAHYDRO-NAPHT-1-YL) ETHYLAMINE

En procédant comme dans la préparation 1, mais en remplaçant l'éthylbenzène par le butylbenzène, on obtient le composé-du titre.

### PREPARATION 6 : (R,S) 2-(5-METHYL-INDOL-3-YL)ETHYLAMINE (d'après Biosci., Biotechnol., Biochem. 1993, 57 (7), pp 1210-11)

### PREPARATIONS 7 A 12 :

En procédant comme dans la préparation 2 mais en utilisant les réactifs appropriés, on obtient les préparations suivantes :

### PREPARATION 7 : 2-(5-PROPYL-BENZO[b]THIOPHEN-3-YL)ETHYLAMINE

### PREPARATION 8 : 2-(5-BUTYL-BENZO[b]THIOPHEN-3-YL)ETHYLAMINE

### PREPARATION 9 : 2-(5-HEXYL-BENZO[b]THIOPHEN-3-YL)ETHYLAMINE

### PREPARATION 10 2-(5-CYCLOPROPYL-BENZO[b]THIOPHEN-3-YL)ETHYLAMINE

### PREPARATION 11 : 2-(5-CYCLOBUTYL-BENZO[b]THIOPHEN-3-YL)ETHYLAMINE

### PREPARATION 12 : 2-(5-CYCLOPROPYLMETHYL-BENZO[b]THIOPHEN-3-YL) ETHYLAMINE

### EXEMPLE 1 : (R,S) N-[2-(7-ETHYL-1,2,3,4-TETRAHYDRONAPHT-1YL)ETHYL] ACETAMIDE

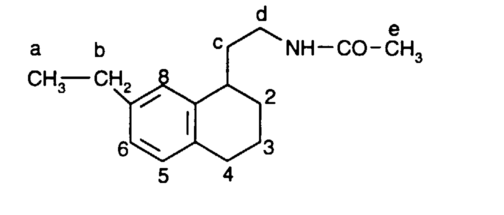

| **Réactifs :** | |
|---|---|
| Chlorhydrate de la (R,S) 2-(7-éthyl-1,2,3,4-tétrahydronapht-1-yl) éthylamine | 0,008 mole (2 g) |
| Chlorure d'acétyle | 0,009 mole (0,7 g) |
| Carbonate de potassium | 0,024 mole (3,3 g) |
| Chloroforme | 20 cm³ |
| Eau : | 10 cm³ |

Dissoudre le chlorhydrate de la (R,S) 2-(7-éthyl-1,2,3,4-tétrahydronapht-1-yl)éthylamine de la préparation 1 dans un mélange eau-chloroforme (10 - 20) puis ajouter 3 équivalents de carbonate de potassium. Refroidir dans un bain de glace-sel. Sous forte agitation magnétique, ajouter goutte à goutte 1,2 équivalents de chlorure d'acétyle. L'agitation est maintenue pendant 45 min.
La phase chloroformique est extraite, lavée par une solution d'acide chlorhydrique 1N puis à l'eau, séchée sur sulfate de magnésium et évaporée à sec.
Le résidu obtenu est purifié par chromatographie.

### Caractéristiques :

245,35 g/mole pour C₁₆H₂₃NO
Huile incolore
Rf = 0,43, éluant : acétone-toluène-cyclohexane (5-3-2)
Rendement : 62 %

| **Analyse spectroscopique dans l'infrarouge :** | |
|---|---|
| 3260 cm⁻¹ | ν NH amide |
| 3060 cm⁻¹ | ν CH aromatiques |
| 2980-2840 cm⁻¹ | ν CH alkyles |
| 1630 cm⁻¹ | ν CO amide |
| 1540 cm⁻¹ | ν C=C aromatiques |

| **Analyse spectroscopique de RMN du proton (300 MHz, DMSO d**_{**6**}**, δ) :** | |
|---|---|
| 1,21 ppm (triplet, 3H) | CH₃ (a), J_{a-b} = 7,60 Hz |
| 1,80 ppm (multiplet, 6H) | CH₂ (2), CH₂ (3) et CH₂ (c) |
| 1,96 ppm (singulet, 3H) | CH₃ (e) |
| 2,58 ppm (quintuplet, 2H) | CH₂ (b), J_{b-a} = 7,60 Hz |
| 2,72 ppm (multiplet, 2H) | CH₂ (4) |
| 2,81 ppm (multiplet, 1H) | CH (1) |
| 3,37 ppm (multiplet, 2H) | CH₂ (d) |
| 5,63 ppm (multiplet, 1H) | NH amide |
| 6,96 ppm (multiplet, 3H) | H aromatiques |

| **Analyse élémentaire :** | | | |
|---|---|---|---|
| Calculé | C : 78,32 % | H : 9,45 % | N : 5,71 % |
| Trouvé | C:77,97 % | H : 9,43 % | N : 5,59 % |

### EXEMPLE 2 : (R,S) N-[2-(7-ETHYL-1,2,3,4-TETRAHYDRONAPHT-1 YL)ETHYL] BUTYRAMIDE

En procédant de la même façon que dans l'exemple 1, mais en remplaçant le chlorure d'acétyle par le chlorure de butyryle, on obtient le composé du titre.

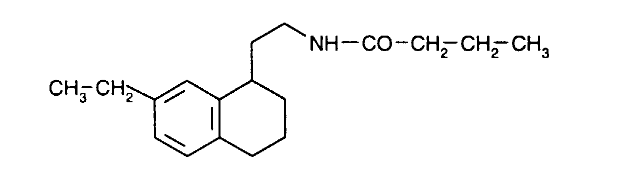

| **Réactifs :** | |
|---|---|
| Chlorhydrate de la (R,S) 2-(7-éthyl-1,2,3,4-tétrahydronapht-1-yl) éthylamine | 0,008 mole (2 g) |
| Chlorure de butyryle | 0,009 mole (1 g) |
| Carbonate de potassium | 0,024 mole (3,3 g) |
| Chloroforme | 20 cm³ |
| Eau | 10 cm³ |

### Caractéristiques :

273,40 g/mole pour C₁₈H₂₇NO
poudre blanche
Température de fusion : 54-56°C
Rendement : 75 %
purifié par chromatographie sur colonne dans l'acétate d'éthyle

### EXEMPLE 3 : (R,S) N-[2-(7-ETHYL-1,2,3,4-TETRAHYDRONAPHT-1YL)ETHYL]CYCLO-PROPANECARBOXAMIDE

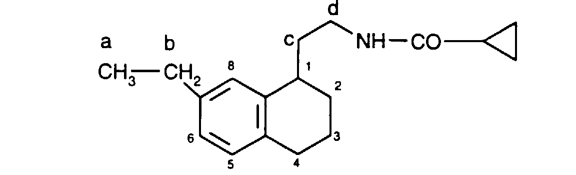

En procédant de la même façon que dans l'exemple 1, mais en remplaçant le chlorure d'acétyle par le chlorure de l'acide cyclopropane carboxylique, on obtient le composé du titre.

| **Réactifs :** | |
|---|---|
| Chlorhydrate de la (R,S) 2-(7-éthyl-1,2,3,4-tétrahydronaphtalène) éthylamine | 0,008 mole (2 g) |
| Chlorure de l'acide cyclopropane carboxylique | 0,009 mole (1 g) |
| Carbonate de potassium | 0,024 mole (3,3 g) |
| Chloroforme | 20 cm³ |
| Eau | 10 cm³ |

### Caractéristiques :

271,39 g/mole pour C₁₈H₂₅NO poudre blanche
Température de fusion : 95-97°C
Rendement : 80%
Solvant de recristallisation : hexane

### EXEMPLE 4 : (R,S) N-[2-(7-ETHYL-1,2,3,4-TETRAHYDRONAPHT-1YL)ETHYL] TRIFLUOROACETAMIDE

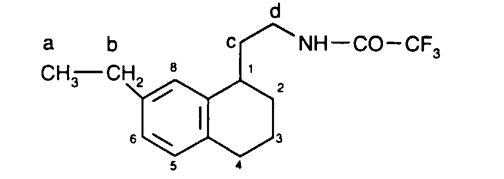

| **Réactifs :** | |
|---|---|
| Chlorhydrate de la (R,S) 2-(7-éthyl-1,2,3,4-tétrahydronapht-1-yl) éthylamine | 0,008 mole (2 g) |
| Anhydride trifluoracétique | 0,009 mole (2 g) |
| Pyridine | 10 cm³ |

Dans une fiole de 50 cm³, dissoudre sous agitation magnétique 2 g du chlorhydrate de la (R,S) 2-(7-éthyl-1,2,3,4-tétrahydronapht-1-yl) éthylamine dans 10 cm³ de pyridine. Refroidir le mélange réactionnel dans de la glace.
Ajouter goutte à goutte 2 g d'anhydride trifluoroacétique. Continuer l'agitation pendant 30 min. Verser dans de la glace. La phase aqueuse est extraite par 3 volumes d'éther. Les phases organiques sont rassemblées, lavées par 3 volumes d'eau, séchées sur sulfate de magnésium puis évaporées sous vide.
Le résidu obtenu est purifé par chromatographie sur colonne puis recristallisé

### Caractéristiques :

299,33 g/mole pour C₁₆H₂₀F₃NO
poudre blanche
Température de fusion : 66-69°C
Rendement: 60 %
purifié par chromatographie sur colonne (éluant-acétate d'éthyle)
Solvant de recristallisation : hexane

| **Analyse spectroscopique dans l'infrarouge :** | |
|---|---|
| 3280 cm⁻¹ | ν NH amide |
| 3070 cm⁻¹ | ν C-H aromatiques |
| 2960-2840 cm⁻¹ | ν C-H alkyles |
| 1630 cm⁻¹ | ν C-O amide |
| 1550 cm⁻¹ | ν C-C aromatiques |

| **Analyse spectroscopique de RMN (300 MHz, CDCl**_{**3**}**, δ) :** | | |
|---|---|---|
| 1,21 ppm (triplet, 3H) | CH₃(a) | J_{a-b} = 7,59 Hz |
| 1,85 ppm (multiplet, 6H) | CH₂(c), CH₂(2) et CH₂(3) | |
| 2,58 ppm (quintuplet, 2H) | CH₂(b) | J_{b-a} = 7,59 Hz |
| 2,73 ppm (multiplet, 2H) | CH₂ (4) | |
| 2,84 ppm (multiplet, 1H) | CH (1) | |
| 3,74 ppm (multiplet, 2H) | CH₂(d) | |
| 6,52 ppm (multiplet, 1H) | NH | |
| 6,97 ppm (multiplet, 3H) | H aromatiques | |

| **Analyse élémentaire :** | | | | |
|---|---|---|---|---|
| Calculé | C : 64,20 % | H : 6,74 % | N : 4,68 % | F : 19.04 % |
| Trouvé | C : 64,13 % | H : 6,70 % | N : 4,62 % | F : 18,78 % |

### EXEMPLE 5 : (R,S) N-[2-(7-ETHYL-1,2,3,4-TETRAHYDRONAPHT-1YL)ETHYL] VALERAMIDE

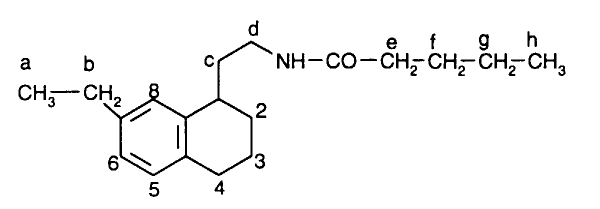

En procédant de la même façon que dans l'exemple 1, mais en remplaçant la chlorure d'acétyle par le chlorure de valéryle, on obtient le composé du titre.

| **Réactifs :** | |
|---|---|
| Chlorhydrate de la (R,S) 2-(7-éthyl-1,2,3,4-tétrahydronapht-1-yl) éthylamine | 0,008 mole (2 g) |
| Chlorure de valéryle | 0,009 mole (1,1 g) |
| Carbonate de potassium | 0,024 mole (3,3 g) |
| Chloroforme | 20 cm³ |
| Eau | 10 cm³ |

### Caractéristiques ;

287,43 g/mole pour C₁₈H₂₉NO
Huile incolore
Rendement : 65 %
purifié par chromatographie sur colonne dans l'acétate d'éthyle

| **Analyse spectroscopique dans l'infrarouge :** | |
|---|---|
| 3290 cm⁻¹ | v NH amide |
| 2990-2820 cm⁻¹ | ν C-H alkyles |
| 1630 cm⁻¹ | ν C=O amide |
| 1530 cm⁻¹ | ν C=C aromatiques |

| **Analyse spectroscopique de RMN (300 MHz, CDCI3, δ) :** | |
|---|---|
| 0,91 ppm (triplet, 3H) | CH₃ (h), J_{h-g} = 7,29 Hz |
| 1,21 ppm (triplet, 3H) | CH₃ (a), J_{a-b} = 7,59 Hz |
| 1,33 ppm (multiplet, 2H) | CH₂ (g) |
| 1,75 ppm (multiplet, 8H) | CH₂ (2), CH₂ (3), CH₂ (c), CH₂ (f) |
| 2,15 ppm (triplet, 2H) | CH₂ (e), J_{e-f} = 7,57 Hz |
| 2,58 ppm (quintuplet, 2H) | CH₂ (b), J_{b-a} = 7,59 Hz |
| 2,71 ppm (multiplet, 2H) | CH₂ (4) |
| 2,80 ppm (multiplet, 1H) | CH (1) |
| 3,37 ppm (multiplet, 2H) | CH₂ (d) |
| 5,71 ppm (multiplet, 1H) | NH |
| 6,97 ppm (multiplet, 3H) | H aromatiques |

### EXEMPLES 6 A 12 :

En procédant de la même façon que dans l'exemple 1, mais en employant le chlorure d'acyle ou l'iso(thio)cyanate approprié, on obtient les exemples suivants :

### EXEMPLE 6: (R,S) N-[2-(7-ETHYL-1,2,3,4-TETRAHYDRONAPHT-1YL)ETHYL]CYCLO-BUTYLCARBOXAMIDE

### EXEMPLE 7: (R,S) N-[2-(7-ETHYL-1,2,3,4-TETRAHYDRONAPHT-1YL)ETHYL] PROPIONAMIDE

### EXEMPLE 8 : (R,S) N-[2-(7-ETHYL-1,2,3,4-TETRAHYDRONAPHT-1YL)ETHYL] ISOBUTYRAMIDE

### EXEMPLE 9: (R,S) N-[2-(7-ETHYL-1,2,3,4-TETRAHYDRONAPHT-1YL)ETHYL] N'-METHYLUREE

### EXEMPLE 10: (R,S) N-[2-(7-ETHYL-1,2,3,4-TETRAHYDRONAPHT-1YL)ETHYL] N'-PROPYLUREE

### EXEMPLE 11 : (R,S) N-[2-(7-ETHYL-1,2,3,4-TETRAHYDRONAPHT-1 YL)ETHYL] N'-CYCLOPROPYLUREE

### EXEMPLE 12 : (R,S) N-[2-(7-ETHYL-1,2,3,4-TETRAHYDRONAPHT-1 YL)ETHYL] N'-CYCLOBUTYLUREE

### EXEMPLE 13: (R,S)N-[2-(5-ETHYL-BENZOTHIOPHEN-3-YL)ETHYL]CYCLOBUTANE-CARBOXAMIDE

En procédant de la même façon que pour la réaction d'amidification de l'exemple 1, mais en remplaçant la 2-(7-éthyl-1,2,3,4-tétrahydronapht-1-yl) éthylamine par la 2-(5-éthyl benzo[b]thiophen-3-yl)éthylamine (préparation 2) et le chlorure d'acétyle par le chlorure de l'acide cyclobutane carboxylique, on obtient le composé du titre.

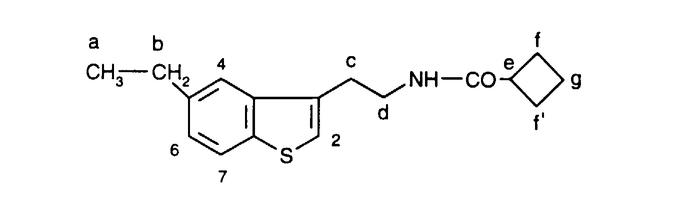

| **Réactifs :** | |
|---|---|
| Chlorhydrate de la 2-(5-éthyl benzo[b]thiophèn-3-yl) éthylamine | 0,001 mole (0,25 g) |
| Carbonate de potassium | 0,003 mole (0,4 g) |
| Chlorure de l'acide cyclobutane carboxylique | 0,0013 mole (0,16 g) |
| chloroforme | 16 cm³ |
| Eau | 8 cm³ |

### Caractéristiques :

286,40 g/mole pour C₁₇H₂₀NOS
poudre blanche
Température de fusion : 105-107°C
Rf = 0,70, éluant : acétone-toluène-cyclohexane (5-3-2)
Solvant de recristallisation : hexane
Rendement : 70 %

| **Analyse spectroscopique dans l'infrarouge :** | |
|---|---|
| 3275 cm⁻¹ | υ NH amide |
| 3070 cm⁻¹ | υ CH aromatique |
| 2980-2840 cm⁻¹ | υ CH alkyles |
| 1630 cm⁻¹ | υ CO amide |
| 1550 cm⁻¹ | υ C=C aromatiques |

| **Analyse spectroscopique de RMN du proton (300 MHz, DMSO d**_{**6**}**, δ) :** | | |
|---|---|---|
| 1,25 ppm (triplet, 3H) | CH₃ (a) | J_{a-b} = 7,53 Hz |
| 1,97 ppm (multiplet, 6H) | CH₂ (f), CH₂ (f') et CH₂ (g) | |
| 2,57 ppm (quintuplet, 2H) | CH₂ (b) | J_{b-a} = 7,53 Hz |
| 2,95 ppm (multiplet, 3H) | CH₂ (c) | |
| 3,36 ppm (multiplet, 2H) | CH₂ (d) | |
| 7,25 ppm (multiplet, 1H) | H₆ | Jₒᵣₜₕₒ =8,27Hz |
| 7,40 ppm (singulet, 1H) | H₂ | |
| 7,68 ppm (multiplet, 1H) | H₄ | |
| 7,85 ppm (multiplet, 2H) | H₇ et NH | |

| **Analyse élémentaire :** | | | | |
|---|---|---|---|---|
| Calculé | C : 71,29 % | H : 7,04 % | N : 4,89 % | Cl : 11,20 % |
| Trouvé | C : 70,88 % | H : 7,32 % | N : 4,90 % | Cl : 11,02 % |

### EXEMPLE 14 : N-[2-(5-ETHYL-BENZOTHIOPHEN-3-YL)ETHYL]ACETAMIDE

En procédant de la même façon que pour la synthèse du composé de l'exemple 14, mais en remplaçant le chlorure de l'acide cyclobutane carboxylique par le chlorure d'acétyle, on obtient le composé du titre.

### Caractéristiques :

247,36 g/mole pour C₁₄H₁₇NOS
température de fusion : 87-88°C

### EXEMPLES 15 A 24 :

En partant de la N-[2-(5-éthyl-benzothiophèn-3-yl)]éthylamine, mais en employant le chlorure d'acide ou l'isocyanate approprié, on obtient les exemples suivants :

### EXEMPLE 15 : N-[2-(5-ETHYL-BENZOTHIOPHEN-3-YL)ETHYL]BUTYRAMIDE

Température de fusion : 62-64°C

### EXEMPLE 16: N-[2-(5-ETHYL-BENZOTHIOPHEN-3-YL)ETHYL]PROPIONAMIDE

Température de fusion : 92-93°C

### EXEMPLE 17: N-[2-(5-ETHYL-BENZOTHIOPHEN-3-YL)ETHYL]VALERAMIDE

Température de fusion : 61-63° C

### EXEMPLE 18: N-[2-(5-ETHYL-BENZOTHIOPHEN-3-YL)ETHYL]CYCLOPROPANE-CARBOXAMIDE

Température de fusion : 92-94° C

### EXEMPLE 19: N-[2-(5-ETHYL-BENZOTHIOPHEN-3-YL)ETHYL]CYCLOHEXANE-CARBOXAMIDE

### EXEMPLE 20 : N-[2-(5-ETHYL-BENZOTHIOPHEN-3-YL)ETHYL] N'-PROPYLUREE

Température de fusion : 137-139° C

### EXEMPLE 21 : N-[2-(5-ETHYL-BENZOTHIOPHEN-3-YL)ETHYL] N'-METHYLUREE

Température de fusion : 133-135° C

### EXEMPLE 22 : N-[2-(5-ETHYL-BENZOTHIOPHEN-3-YL)ETHYL] N'-ETHYLUREE

### EXEMPLE 23 : N-[2-(5-ETHYL-BENZOTHIOPHEN-3-YL)ETHYL] N'-CYCLOPROPYL UREE

### EXEMPLE 24 : N-[2-(5-ETHYL-BENZOTHIOPHEN-3-YL)ETHYL] N'-CYCLOHEXYLUREE

### EXEMPLE 25 : N-[2-(5-ETHYL-BENZOTHIOPHEN-3-YL)ETHYL]TRIFLUORO ACETAMIDE

### EXEMPLE 26 : N-[2-(5-METHYL-INDOL-3-YL)ETHYL]ACETAMIDE

En procédant de la même façon que pour la réaction d'amidification de l'exemple 1, en employant comme réactifs la 5-méthyl tryptamine (préparation 6) et le chlorure d'acétyle, on obtient le composé du titre.

### EXEMPLES 27 A 31 :

En partant de la 5-méthyl-tryptamine mais en employant le chlorure d'acyle ou l'isocyanate approprié, on obtient les exemples suivants :

### EXEMPLE 27 : N-[2-(5-METHYL-INDOL-3-YL)ETHYL]CYCLOPROPANECARBOXAMIDE

### EXEMPLE 28 : N-[2-(5-METHYL-INDOL-3-YL)ETHYL]BUTYRAMIDE

### EXEMPLE 29 : N-[2-(5-METHYL-INDOL-3-YL)ETHYL] TRIFLUOROACETAMIDE

### EXEMPLE 30 : N-[2-(5-METHYL-INDOL-3-YL)ETHYL] N'-METHYLUREE

### EXEMPLE 31: N-[2-(5-METHYL-INDOL-3-YL)ETHYL] N'-PROPYLUREE

### EXEMPLE 32 : (R,S) N-[2-(7-METHYL-1,2,3,4-TETRAHYDRONAPHT-1-YL)ETHYL] ACETAMIDE

En procédant de la même façon que pour la réaction d'amidification du composé de l'exemple 1, mais en employant comme réactifs la (R,S) 2-(7-méthyl-1,2,3,4-tétrahydronapht-1-yl)éthylamine (préparation 3) et le chlorure d'acétyle, on obtient le composé du titre.

### EXEMPLES 33 A 36 :

En partant de la 2-(7-méthyl-1,2,3,4-tétrahydronapht-1-yl)éthylamine, mais en employant le chlorure d'acyle ou l'isocyanate approprié, on obtient les exemples suivants :

### EXEMPLE 33 : (R,S) N-[2-(7-METHYL-1,2,3,4-TETRAHYDRONAPHT-1-YL)ETHYL] CYCLOPROPANECARBOXAMIDE

### EXEMPLE 34 : (R,S) N-[2-(7-METHYL-1,2,3,4-TETRAHYDRONAPHT-1-YL)ETHYL] N'-METHYLUREE

### EXEMPLE 35 : (R,S) N-[2-(7-METHYL-1,2,3,4-TETRAHYDRONAPHT-1-YL)ETHYL] N'-PROPYLUREE

### EXEMPLE 36 : (R,S) N-[2-(7-METHYL-1,2,3,4-TETRAHYDRONAPHT-1-YL)ETHYL] BUTYRAMIDE

### EXEMPLE 37 : (R,S) N-[2-(7-PROPYL-1,2,3,4-TETRAHYDRONAPHT-1-YL)ETHYL] ACETAMIDE

En procédant de la même façon que lors de la réaction d'amidification du composé de l'exemple 1, mais en employant comme réactif la (R,S) 2-(7-propyl-1,2,3,4-tétrahydronapht-1-yl)éthylamine (préparation 4), on obtient le composé du titre.

### EXEMPLES 38 A 41 :

En partant de la (R,S) 2-(7-propyl-1,2,3,4-tétrahydronapht-1-yl)éthylamine, mais en employant le chlorure d'acide ou l'isocyanate approprié, on obtient les exemples suivants :

### EXEMPLE 38 : (R,S) N-[2-(7-PROPYL-1,2,3,4-TETRAHYDRONAPHT-1-YL)ETHYL] CYCLOPROPANECARBOXAMIDE

### EXEMPLE 39 : (R,S) N-[2-(7-PROPYL-1,2,3,4-TETRAHYDRONAPHT-1-YL)ETHYL] N'-METHYLUREE

### EXEMPLE 40 : (R,S) N-[2-(7-PROPYL-1,2,3,4-TETRAHYDRONAPHT-1-YL)ETHYL] N'-PROPYLUREE

### EXEMPLE 41 : (R,S) N-[2-(7-PROPYL-1,2,3,4-TETRAHYDRONAPHT-1-YL)ETHYL] TRIFLUOROACETAMIDE

### EXEMPLE 42 : (R,S) N-[2-(7-BUTYL-1,2,3,4-TETRAHYDRO-NAPHT-1-YL)ETHYL] ACETAMIDE

En procédant de la même façon que pour la réaction d'amidification du composé de l'exemple 1, mais en employant comme réactif la 2-(7-butyl-napht-1-yl)éthylamine (préparation 5), on obtient le composé du titre.

### EXEMPLES 43 A 46 :

En partant de la 2-(7-butyl-1,2,3,4-tétrahydronapht-1-yl)éthylamine, mais en employant le chlorure d'acide ou l'isocyanate approprié, on obtient les exemples suivants :

### EXEMPLE 43 : (R,S) N-[2-(7-BUTYL-1,2,3,4-TETRAHYDRONAPHT-1-YL)ETHYL] N'-METHYLUREE

### EXEMPLE 44 : (R,S) N-[2-(7-BUTYL-1,2,3,4-TETRAHYDRONAPHT-1-YL)ETHYL] N'-PROPYLUREE

### EXEMPLE 45 : (R,S) N-[2-(7-BUTYL-1,2,3,4-TETRAHYDRONAPHT-1-YL)ETHYL] CYCLOPROPANECARBOXAMIDE

### EXEMPLE 46 : (R,S) N-[2-(7-BUTYL-1,2,3,4-TETRAHYDRONAPHT 1YL)ETHYL] TRIFLUOROACETAMIDE

### EXEMPLES 47 A 52 :

En utilisant les préparations 1 et 2 mais en employant les isothiocyanates appropriés, on obtient les composés des exemples suivants :

### EXEMPLE 47 : (R,S) N-[2-(7-ETHYL-1,2,3,4-TETRAHYDRONAPHT-1-YL)ETHYL] N'-METHYLTHIOUREE

### EXEMPLE 48 :- (R,S) N-[2-(7-ETHYL-1,2,3,4-TETRAHYDRONAPHT-1-YL)ETHYL] N'-ETHYL-THIOUREE

### EXEMPLE 49: (R,S) N-[2-(7-ETHYL-1,2,3,4-TETRAHYDRONAPHT-1-YL)ETHYL] N'-PROPYL-THIOUREE

### EXEMPLE 50 : N-[2-(5-ETHYL-BENZOTHIOPHEN-3-YL)ETHYL] N'-METHYLTHIOUREE

### EXEMPLE 51 : N-[2-(5-ETHYL-BENZOTHIOPHEN-3-YL)ETHYL] N'-ETHYLTHIOUREE

### EXEMPLE 52 : N-[2-(5-ETHYL-BENZOTHIOPHEN-3-YL)ETHYL] N'-PROPYLTHIOUREE

### EXEMPLES 53 A 100 :

En procédant comme décrit précédemment mais en partant des préparations 7 à 12, on obtient les composés des exemples suivants :

### EXEMPLE 53 : N-[2-(5-PROPYL-BENZOTHIOPHEN-3-YL)ETHYL]ACETAMIDE

### EXEMPLE 54 : N-[2-(5-PROPYL-BENZOTHIOPHEN-3-YL)ETHYL]PROPIONAMIDE

### EXEMPLE 55 : N-[2-(5-PROPYL-BENZOTHIOPHEN-3-YL)ETHYL]BUTYRAMIDE

### EXEMPLE 56 : N-[2-(5-PROPYL-BENZOTHIOPHEN-3-YL)ETHYL]VALERAMIDE

### EXEMPLE 57 : N-[2-(5-PROPYL-BENZOTHIOPHEN-3-YL)ETHYL]CYCLOPROPANE-CARBOXAMIDE

### EXEMPLE 58 : N-[2-(5-PROPYL-BENZOTHIOPHEN-3-YL)ETHYL]CYCLOBUTANE-CARBOXAMIDE

### EXEMPLE 59 : N-[2-(5-PROPYL-BENZOTHIOPHEN-3-YL)ETHYL]N'-METHYLUREE

### EXEMPLE 60 : N-[2-(5-PROPYL-BENZOTHIOPHEN-3-YL)ETHYL]N'-PROPYLUREE

### EXEMPLE 61 : N-[2-(5-BUTYL-BENZOTHIOPHEN-3-YL)ETHYL]ACETAMIDE

### EXEMPLE 62 : N-[2-(5-BUTYL-BENZOTHIOPHEN-3-YL)ETHYL]PROPIONAMIDE

### EXEMPLE 63 : N-[2-(5-BUTYL-BENZOTHIOPHEN-3-YL)ETHYL]BUTYRAMIDE

### EXEMPLE 64 : N-[2-(5-BUTYL-BENZOTHIOPHEN-3-YL)ETHYL]VALERAMIDE

### EXEMPLE 65 : N-[2-(5-BUTYL-BENZOTHIOPHEN-3-YL)ETHYL]CYCLOPROPANE-CARBOXAMIDE

### EXEMPLE 66 : N-[2-(5-BUTYL-BENZOTHIOPHEN-3-YL)ETHYL]CYCLOBUTANE-CARBOXAMIDE

### EXEMPLE 67 : N-[2-(5-BUTYL-BENZOTHIOPHEN-3-YL)ETHYL]N'-METHYLUREE

### EXEMPLE 68 : N-[2-(5-BUTYL-BENZOTHIOPHEN-3-YL)ETHYL]N'-PROPYLUREE

### EXEMPLE 69 : N-[2-(5-HEXYL-BENZOTHIOPHEN-3-YL)ETHYL]ACETAMIDE

### EXEMPLE 70 : N-[2-(5-HEXYL-BENZOTHIOPHEN-3-YL)ETHYL]PROPIONAMIDE

### EXEMPLE 71 : N-[2-(5-HEXYL-BENZOTHIOPHEN-3-YL)ETHYL]BUTYRAMIDE

### EXEMPLE 72 : N-[2-(5-HEXYL-BENZOTHIOPHEN-3-YL)ETHYL]VALERAMIDE

### EXEMPLE 73 : N-[2-(5-HEXYL-BENZOTHIOPHEN-3-YL)ETHYL]CYCLOPROPANE-CARBOXAMIDE

### EXEMPLE 74 : N-[2-(5-HEXYL-BENZOTHIOPHEN-3-YL)ETHYL]CYCLOBUTANE-CARBOXAMIDE

### EXEMPLE 75 : N-[2-(5-HEXYL-BENZOTHIOPHEN-3-YL)ETHYL]N'-METHYLUREE

### EXEMPLE 76 : N-[2-(5-H EXYL-BENZOTHIOPHEN-3-YL)ETHYL]N'-PROPYLUREE

### EXEMPLE 77: N-[2-(5-CYCLOPROPYL-BENZOTHIOPHEN-3-YL)ETHYL]ACETAMIDE

### EXEMPLE 78: N-[2-(5-CYCLOPROPYL-BENZOTHIOPHEN-3-YL)ETHYL]PROPIONAMIDE

### EXEMPLE 79: N-[2-(5-CYCLOPROPYL-BENZOTHIOPHEN-3-YL)ETHYL]BUTYRAMIDE

### EXEMPLE 80 : N-[2-(5-CYCLOPROPYL-BENZOTHIOPHEN-3-YL)ETHYL]VALERAMIDE

### EXEMPLE 81 : N-[2-(5-CYCLOPROPYL-BENZOTHIOPHEN-3-YL)ETHYL]CYCLOPROPANECARBOXAMIDE

### EXEMPLE 82 : N-[2-(5-CYCLOPROPYL-BENZOTHIOPHEN-3-YL)ETHYL]CYCLO-BUTANECARBOXAMIDE

### EXEMPLE 83: N-[2-(5-CYCLOPROPYL-BENZOTHIOPHEN-3-YL)ETHYL]N'-METHYLUREE

### EXEMPLE 84 : N-[2-(5-CYCLOPROPYL-BENZOTHIOPHEN-3-YL)ETHYL]N'-PROPYLUREE

### EXEMPLE 85: N-[2-(5-CYCLOBUTYL-BENZOTHIOPHEN-3-YL)ETHYL]ACETAMIDE

### EXEMPLE 86 : N-[2-(5-CYCLOBUTYL-BENZOTHIOPHEN-3-YL)ETHYL]PROPIONAMIDE

### EXEMPLE 87: N-[2-(5-CYCLOBUTYL-BENZOTHIOPHEN-3-YL)ETHYL]BUTYRAMIDE

### EXEMPLE 88: N-[2-(5-CYCLOBUTYL-BENZOTHIOPHEN-3-YL)ETHYL]VALERAMIDE

### EXEMPLE 89 : N-[2-(5-CYCLOBUTYL-BENZOTHIOPHEN-3-YL)ETHYL]CYCLO-PROPANECARBOXAMIDE

### EXEMPLE 90 : N-[2-(5-CYCLOBUTYL-BENZOTHIOPHEN-3-YL)ETHYL]CYCLO-BUTANECARBOXAMIDE

### EXEMPLE 91 : N-[2-(5-CYCLOBUTYL-BENZOTHIOPHEN-3-YL)ETHYL]N'-METHYLUREE

### EXEMPLE 92: N-[2-(5-CYCLOBUTYL-BENZOTHIOPHEN-3-YL)ETHYL]N'-PROPYLUREE

### EXEMPLE 93 : N-[2-(5-CYCLOPROPYLMETHYL-BENZOTHIOPHEN-3-YL)ETHYL] ACETAMIDE

### EXEMPLE 94 : N-[2-(5-CYCLOPROPYLMETHYL-BENZOTHIOPHEN-3-YL)ETHYL] PROPIONAMIDE

### EXEMPLE 95 : N-[2-(5-CYCLOPROPYLMETHYL-BENZOTHIOPHEN-3-YL)ETHYL] BUTYRAMIDE

### EXEMPLE 96: N-[2-(5-CYCLOPROPYLMETHYL-BENZOTHIOPHEN-3-YL)ETHYL] VALERAMIDE

### EXEMPLE 97: N-[2-(5-CYCLOPROPYLMETHYL-BENZOTHIOPHEN-3-YL)ETHYL] CYCLOPROPANECARBOXAMIDE

### EXEMPLE 98 : N-[2-(5-CYCLOPROPYLMETHYL-BENZOTHIOPHEN-3-YL)ETHYL] CYCLOBUTANECARBOXAMIDE

### EXEMPLE 99: N-[2-(5-CYCLOPROPYLMETHYL-BENZOTHIOPHEN-3-YL)ETHYL]N'-METHYLUREE

### EXEMPLE 100 : N-[2-(5-CYCLOPROPYLMETHYL-BENZOTHIOPHEN-3-YL)ETHYL]N'-PROPYLUREE

### EXEMPLE 101: N-[2-(7-ETHYL-NAPHT-1-YL)ETHYL]ACETAMIDE

### EXEMPLE 102 : N-[2-(7-ETHYL-NAPHT-1-YL)ETHYL]CYCLOPROPANECARBOXAMIDE

### EXEMPLE 103: N-[2-(7-ETHYL-NAPHT-1-YL)ETHYL]CYCLOBUTANE CARBOXAMIDE

### EXEMPLE 104 : N-[2-(7-ETHYL-NAPHT-1-YL)ETHYL]TRIFLUOROACETAMIDE

### EXEMPLE 105 : N-[2-(7-ETHYL-NAPHT-1 -YL)ETHYL]N'-METHYLUREE

### EXEMPLE 106 : N-[2-(7-ETHYL-NAPHT-1 -YL)ETHYL]N'-PROPYLUREE

### EXEMPLE 107 : N-[2-(7-METHYL-NAPHT-1-YL)ETHYL]ACETAMIDE

### EXEMPLE 108 : N-[2-(7-PROPYL-NAPHT-1 -YL)ETHYL]ACETAMIDE

### EXEMPLE 109 : N-[2-(7-BUTYL-NAPHT-1 -YL)ETHYL]ACETAMIDE

### EXEMPLE 110 : N-[2-(7-HEXYL-NAPHT-1-YL)ETHYL]ACETAMIDE

### ETUDE PHARMACOLOGIQUE

### EXEMPLE A : ETUDE DE LA TOXICITE AIGUE

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée.

La DL 50 des produits testés est supérieure à 1000 mg.kg⁻¹ pour les composés étudiés ce qui indique la faible toxicité des composés de l'invention.

### EXEMPLE B : ETUDE DE LIAISON AUX RECEPTEURS DE LA MELATONINE

### B1) ETUDE SUR DES CELLULES DE LA PARS TUBERALIS DE MOUTON

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology vol. (1), pp 1-4 (1989)).

### PROTOCOLE

1) Les membranes de pars Tuberalis de mouton sont préparées et utilisées comme tissu cible dans des expériences de saturation pour déterminer les capacités et affinités de liaison pour la 2-[¹²⁵I]- iodomélatonine.
2) Les membranes de Pars tuberalis de mouton sont utilisées comme tissu cible, avec les différents composés à tester, dans des expériences de liaison compétitive par rapport à la 2-[¹²⁵I]- mélatonine.

Chaque expérience est réalisée en triple et une gamme de concentrations différentes est testée pour chaque composé.

Les résultats permettent de déterminer, après traitement statistique, les affinités de liaison du composé testé.

### RESULTATS

Il apparaît que les composés de l'invention possèdent une puissante affinité pour les récepteurs de la mélatonine supérieure à la mélatonine elle-même.

### B2) ETUDE SUR DES MEMBRANES DE CELLULES DU CERVEAU DE POULET (GALLUS DOMESTICUS)

Les animaux utilisés sont des poulets (Gallus domesticus) agés de 12 jours. Ils sont sacrifiés entre 13 et 17 heures le jour de leur arrivée. Les cerveaux sont rapidement prélevés et congelés à - 200°C puis conservés à - 80°C. Les membranes sont préparées selon la méthode décrite par Yuan et Pang (Journal of Endocrinology 128, pages 475-482, 1991). La 2-[¹²⁵I] mélatonine est incubée en présence des membranes dans une solution tamponnée à pH 7.4 pendant 60 min à 25°C. A l'issue de cette période, la suspension membranaire est filtrée (Whatman GF/C). La radioactivité retenue sur le filtre est déterminée à l'aide d'un compteur à scintillation liquide Beckman® LS 6000.

Les produits utilisés sont :
- 2-[¹²⁵I] mélatonine
- mélatonine
- produits courants
- molécules originales

En screening primaire, les molécules sont testées à 2 concentrations (10⁻⁷ et 10⁻⁵ M). Chaque résultat est la moyenne de n=3 mesures indépendantes. Les molécules actives retenues d'après les résultats du screening primaire ont fait l'objet d'une détermination quantitative de leur efficacité (IC₅₀). Elles sont utilisées à 10 concentrations différentes.

Ainsi les valeurs d'IC₅₀ trouvées pour les composés préférés de l'invention, qui correspondent aux valeurs de l'affinité montrent que la liaison des composés de l'invention testés est très puissante.

### EXEMPLE C : TEST DES QUATRE PLAQUES

Les produits de l'invention sont administrés par voie oesophagienne à des lots de dix souris. Un lot reçoit du sirop de gomme. 30 minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des composés de l'invention.

### EXEMPLE D: COMPOSES DE L'INVENTION SUR LES RYTHMES CIRCADIENS D'ACTIVITE LOCOMOTRICE DU RAT

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et en particulier sur les rythmes circadiens d'activité locomotrice qui représentent un marqueur fiable de l'activité de l'horloge circadienne endogène.

Dans cette étude, on évalue les effets de telles molécules sur un modèle expérimental particulier, à savoir le rat placé en isolement temporel (obscurité permanente).

### PROTOCOLE EXPERIMENTAL

Des rats mâles Long Evans âgés de un mois sont soumis dès leur arrivée au laboratoire à un cycle lumineux de 12h de lumière par 24h (LD 12 : 12).

Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule ; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### RESULTATS :

II apparaît clairement que les composés de l'invention permettent d'agir de façon puissante sur le rythme circadien via le système mélatoninergique.

### EXEMPLE E : ACTIVITE ANTIARYTHMIQUE

### PROTOCOLE

(Ref : LAWSON J.W. et al. J. Pharmacol. Expert. Therap. 160 : 22-31, 1968)

La substance testée est administrée en intrapéritonéal à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE F : ACTIVITE ANTI-AGREGANTE PLAQUETTAIRE

### PROTOCOLE

(Ref. : Bertele V. et al. Science. 220 : 517-519, 1983
Ibid, Eur. J. Pharmacol. 85 : 331-333, 1982)

Les composés de l'invention (100 µg/ml) sont testés pour leur capacité d'inhiber l'agrégation plaquettaire irréversible induite par l'arachidonate de sodium (50 µg/ml) dans du plasma de lapin enrichi en plaquettes.

Une inhibition de plus de 50 % de l'agrégation maximum indique une activité significative pour les composés de l'invention.

Ce test *in vitro* montre que les composés de l'invention sont de bons candidats pour le traitement des maladies cardiovasculaires, notamment les thromboses

### EXEMPLE G : PROLONGATION DU TEMPS DE SAIGNEMENT

### PROTOCOLE

(Ref. : Djana E. et al. Thrombosis Research. 15: 191-197, 1979)
Butler K.D. et al. Thromb. Haemostasis. 47 : 46-49, 1982)

Les composés à tester sont administrés par voie orale (100 mg/kg) à un groupe de 5 souris 1h avant le sectionnement standardisé du bout de chaque queue (0,5 mm).

Les souris sont immédiatement suspendues verticalement, les queues étant immergées de 2 cm dans un tube à essai contenant une solution saline isotonique à 37°C.

Le temps requis pour que le saignement cesse pendant une période de 15 secondes est alors déterminé.

Une prolongation de plus de 50 % du temps de saignement relative à un groupe d'animaux contrôle est considérée comme significative pour les composés de l'invention.

Ce test *in vivo* confirme l'intérêt des composés de l'invention pour le traitement des pathologies cardiovasculaires puisque les composés de l'invention prolongent le temps de saignement.

### EXEMPLE H : TEST D'HYPOXIE HYPOBARE

### PROTOCOLE

(Ref. : Gotti B., et Depoortere H., Circ. Cerebrale, Congrès de Circulation Cérébrale, Toulouse, 105-107, 1979)

Les composés à tester sont administrés par voie intrapéritonéale (100 mg/kg) à un groupe de 3 souris 30 minutes avant d'être placés dans une chambre à la pression hypobare de 20 cm Hg.

La prolongation du temps de survie par rapport à un groupe d'animaux traités avec le véhicule de plus de 100 % en absence d'effet dépresseur du système nerveux central indique une activité cérébroprotective des composés de l'invention.

### EXEMPLE I : COMPOSITION PHARMACEUTIQUE : COMPRIMES

| 1000 comprimés dosés à 5 mg de N-[2-(5-éthyl-benzothiophèn-3-yl)éthyl]acétamide | |
|---|---|
| N-[2-(5-éthyl-benzothiophèn-3-yl)éthyl]acétamide | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I): 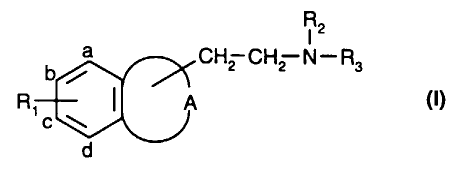 dans laquelle :
- R₁ représente un radical choisi parmi alkyle, alkyle substitué, cycloalkyle, cycloalkyle-substitué, cycloalkylalkyle, et cycloalkylalkyle substitué,
- A forme avec le noyau benzènique auquel il est lié un groupement cyclique choisi parmi tétrahydronaphtalène, dihydronaphtalène, naphtalène, benzothiophène, 2,3-dihydrobenzothiophène, indoline, indoline substitué, indole et indole substitué,
- R₂ représente un hydrogène ou un alkyle,
- R₃ représente :
• un groupement R₃₁ : 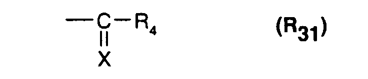 avec X représentant un soufre ou un oxygène et R₄ représentant un hydrogène ou un radical R₄₁ choisi parmi alkyle, alkyle substitué, alcényle, alcynyle, cycloalkyle, cycloalkyle substitué, cycloalkylalkyle et cycloalkylalkyle substitué,
• ou un groupement de formule (R₃₂) : 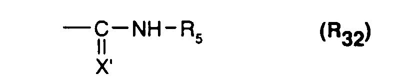 avec X' représentant un soufre ou un oxygène et R₅ représentant un hydrogène ou un radical choisi parmi alkyle, alkyle substitué, cycloalkyle, cycloalkyle substitué, cycloalkylalkyle et cycloalkylalkyle substitué,
étant entendu que lors de la description de la formule (I), et sauf mention contraire :
- les termes "alkyle" et "alkoxy" désignent des groupements linéaires ou ramifiés contenant de 1 à 6 atomes de carbone,
- les termes "alcényle" et "alcynyle" désignent des groupements linéaires ou ramifiés contenant de 2 à 6 atomes,
- le terme "cycloalkyle" désigne un groupement de 3 à 8 atomes de carbone,
- le terme "substitué" associé au radical alkyle signifie que ce radical est substitué par un ou plusieurs substituants choisis parmi halogène, alkyle, hydroxy et alkoxy,
- le terme "substitué" associé au radical "cycloalkyle" et "cycloalkylalkyle" signifie que ce radical est substitué par un ou plusieurs radicaux ou groupements choisis parmi halogène, alkyle et oxo,
- le terme "substitué" associé aux termes "indole" et "indoline" signifie que ces groupements sont substitués sur l'azote en position 1 par un radical choisi parmi -Ra, -CO-Ra et -CO-O-Ra dans lequel Ra représente un radical alkyle, phényle ou phénylalkyle,
et leurs énantiomères et diastéréoisomères.

2. Composés de formule (I) selon la revendication 1 dans laquelle, pris séparément ou ensemble,
- R₁ représente un alkyle,
- R₁ représente un (C₂-C₆) alkyle,
- R₁ représente un éthyle,
- R₁ représente un propyle,
- R₁ représente un butyle,
- A forme avec le noyau benzénique auquel il est lié un tétrahydronaphtalène,
- A forme avec le noyau benzènique auquel il est lié un naphtalène,
- A forme avec le noyau benzènique auquel il est lié un dihydronaphtalène,
- A forme avec le noyau benzènique auquel il est lié un benzothiophène,
- A forme avec le noyau benzènique auquel il est lié un indole,
- A forme avec le noyau benzènique auquel il est lié un indole substitué,
- R₂ représente un hydrogène,
- R₂ représente un alkyle,
- R₃ représente un groupement R₃₁ tel que défini dans la formule (I),
- R₃ représente un groupement R₃₂ tel que défini dans la formule (I),
- R₄ représente un atome d'hydrogène,
- R₄ représente un alkyle,
- R₄ représente un cycloalkyle,
- R₄ représente un alcényle,
- R₅ représente un hydrogène,
- R₅ représente un alkyle,
- R₅ représente un cycloalkyle,
- X représente un oxygène,
- X représente un soufre,
- X' représente un oxygène,
- ou X' représente un soufre.

3. Composés de formule (I) selon la revendication 1 répondant aux formules respectives (1) à (5) :

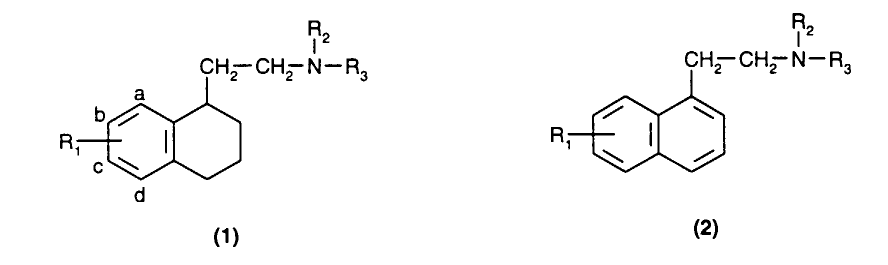

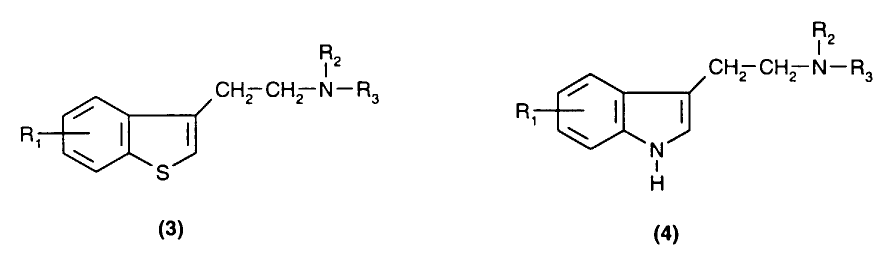

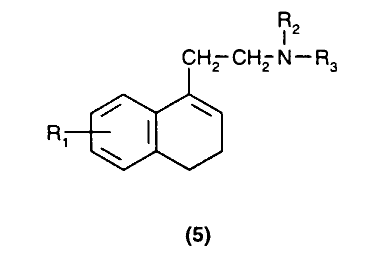

4. Composés selon la revendication 1 qui sont:
- N-[2-(5-éthyl benzothiophèn-3-yl)éthyl]acétamide,
- N-[2-(5-éthyl benzothiophèn-3-yl)éthyl]cyclobutanecarboxamide,
- N-[2-(7-éthyl-1,2,3,4-tétrahydronapht-1-yl)éthyl]acétamide,
- N-[2-(7-éthyl-1,2,3,4-tétrahydronapht-1-yl)éthyl]butyramide,
- N-[2-(7-éthyl-1,2,3,4-tétrahydronapht-1-yl)éthyl]cyclopropanecarboxamide,
- N-[2-(7-éthyl-1,2,3,4-tétrahydronapht-1-yl)éthyl]pentanamide,
- et le N-[2-(7-éthyl-1,2,3,4-tétrahydronapht-1-yl)éthyl]trifluoroacétamide.

5. Procédé de préparation des composés de formule (I) caractérisé en ce que : on fait réagir un composé de formule (II) : 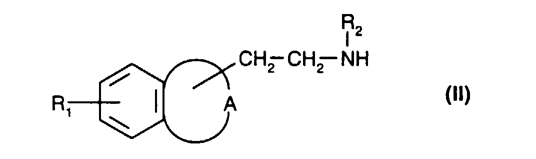 dans laquelle R₁ et A sont tels que définis dans la revendication 1,
- soit avec l'acide formique ou avec un composé de formule (IIIa) ou (IIIb) : 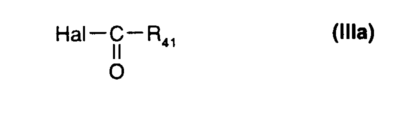 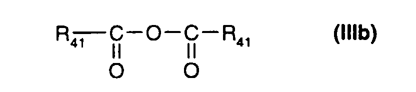 dans laquelle R₄₁ est tel que défini dans la revendication 1 et Hal représente un halogène, afin d'obtenir les composés de formule (I/a): 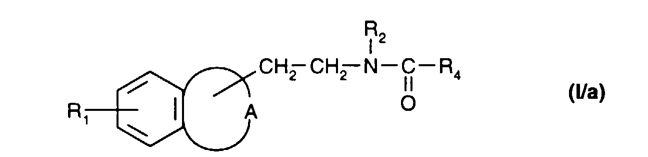 dans laquelle R₁, R₂, R₄ et A sont tels que définis précédemment,
composés de formule (I/a) soumis au réactif de Lawesson pour obtenir les composés de formule (I/a'): 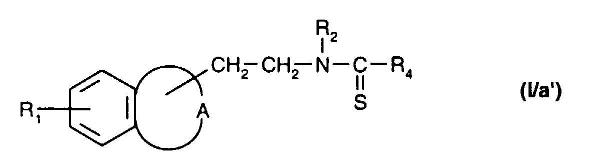 dans laquelle R₁, R₂, R₄ et A sont tels que définis précédemment,
- soit avec un composé de formule (IV) :
X'=C=N-R₅ **(IV)**
dans laquelle X' et R₅ sont tels que définis dans la revendication 1 afin d'obtenir les composés de formule (I/b) : 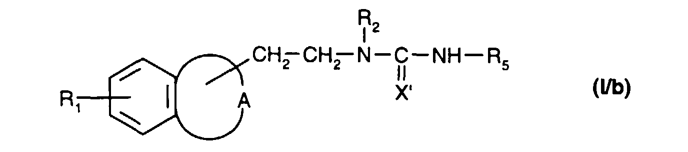 dans laquelle R₁, R₂, R₅, A et X' sont tels que définis précédemment,
les composés de formule (I/a), (I/a') et (I/b) formant l'ensemble des composés de formule (I) selon la revendication 1, composés de formule (I) qui sont, le cas échéant, séparés en leurs différents énantiomères ou diastéréoisomères.

6. Procédé de préparation selon la revendication 5 des composés de formule (I/c): 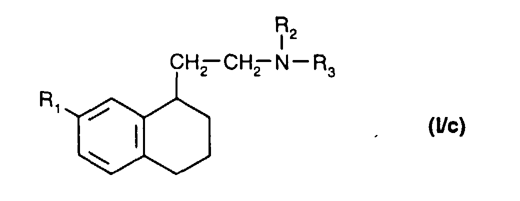 dans laquelle R₁, R₂ et R₃ sont tels que définis dans la revendication 1, caractérisé en ce que : on fait réagir un composé de formule (Il/a) : 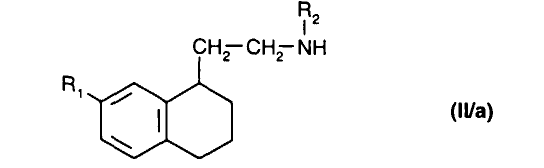 dans laquelle R₁ et R₂ sont tels que définis précédemment,
- soit avec un composé de formule (IIIa) ou (IIIb) tels que définis dans la revendication 5,
afin d'obtenir les composés de formule (I/d) : 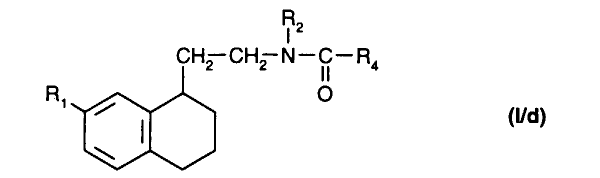 dans laquelle R₁, R₂ et R₄ sont tels que définis précédemment, qui sont ensuite soumis au réactif de Lawesson pour obtenir les composés de formule (I/d') : 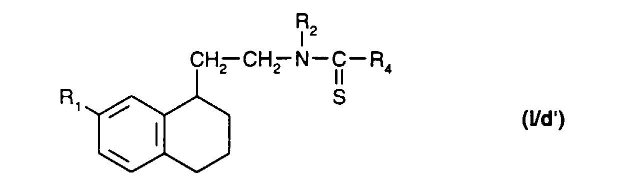 dans laquelle R₁, R₂, et R₄ sont tels que définis précédemment,
- soit avec un composé de formule (IV) tel que défini précédemment, afin d'obtenir les composés de formule (I/e) : 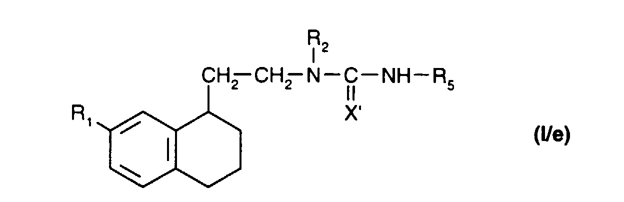 dans laquelle R₁, R₂, R₅ et X' sont tels que définis précédemment,
les composés de formule (I/d), (I/d') et (I/e) formant l'ensemble des composés de formule (I/c),
les composés de formule (I/c) pouvant être séparés en leurs différents énantiomères ou diastéréoisomères.

7. Procédé de préparation des composés de formule (I/f): 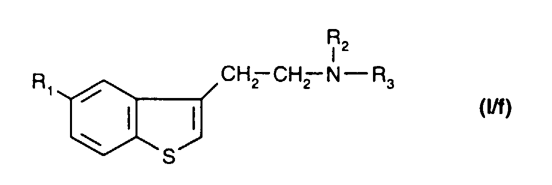 dans laquelle R₁, R₂ et R₃ sont tels que définis dans la revendication 1, caractérisé en ce que: on fait réagir un composé de formule (II/b) ; 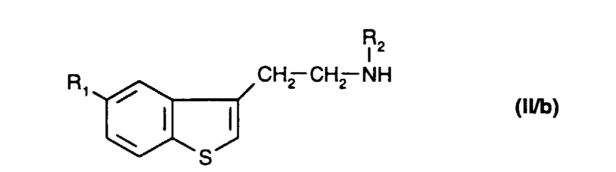 dans laquelle R₁ et R₂ sont tels que définis précédemment,
- soit avec un composé de formule (IIIa) ou (IIIb) tels que définis dans la revendication 5, afin d'obtenir les composés de formule (I/g): 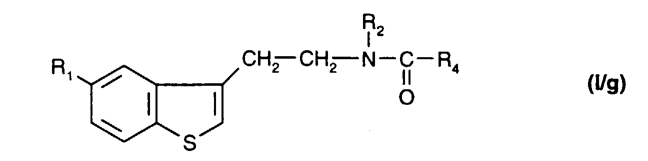 dans laquelle R₁, R₂ et R₄ sont tels que définis précédemment, qui sont ensuite soumis au réactif de Lawesson pour obtenir les composés de formule (I/g'): 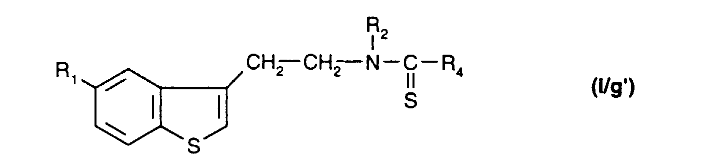 dans laquelle R₁, R₂ et R₄ sont tels que définis précédemment,
- soit avec un composé de formule (IV) tel que défini précédemment, afin d'obtenir les composés de formule (I/h): 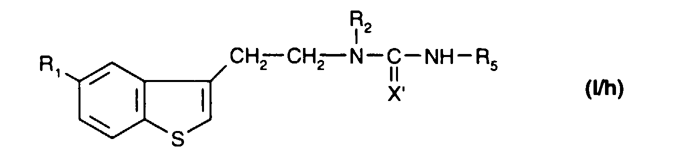 dans laquelle R₁, R₂, R₅ et X' sont tels que définis précédemment,
les composés de formule (I/g), (I/g') et (I/h) formant l'ensemble des composés de formule (I/f),
les composés de formule (I/f) pouvant être séparés en leurs différents énantiomères ou diastéréoisomères.

8. Compositions pharmaceutiques contenant les produits de formule (I) selon la revendication 1 en combinaison avec un ou plusieurs excipients pharmaceutiquement acceptables.

9. Compositions pharmaceutiques selon la revendication 8 utiles pour le traitement des troubles mélatoninergiques.

10. Compositions pharmaceutiques selon la revendication 8 utiles dans les traitements des dépressions saisonnières, des troubles du sommeil, des pathologies cardiovasculaires, des insomnies et fatigues dues aux décalages horaires, des troubles de l'appétit et de l'obésité.

## Patentansprüche

1. Verbindungen der Formel (I): 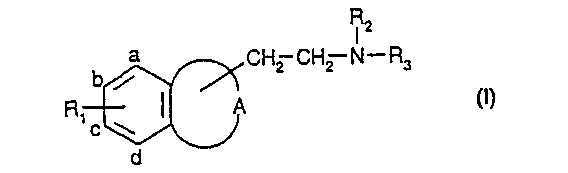 in der:
- R₁ eine Gruppe ausgewählt aus Alkyl, substituiertem Alkyl, Cycloalkyl, substituiertem Cycloalkyl, Cycloalkylalkyl und substituiertem Cycloalkylalkyl,
- A zusammen mit dem Benzolkern, an den es gebunden ist, eine cyclische Gruppe ausgewählt aus Tetrahydronaphthalin, Dihydronaphthalin, Naphthalin, Benzothiophen, 2,3-Dihydrobenzothiophen, Indolin, substituiertem Indolin, Indol und substituiertem Indol,
- R₂ Wasserstoff oder eine Alkylgruppe,
- R₃:
• eine Gruppe R₃₁ : 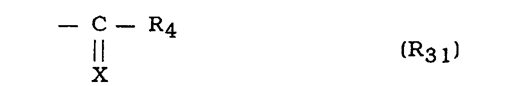 in der X Schwefel oder Sauerstoff und R₄ Wasserstoff oder eine Gruppe R₄₁ ausgewählt aus Alkyl, substituiertemAlkyl, Alkenyl, Alkinyl, Cycloalkyl, substituiertem Cycloalkyl, Cycloalkylalkyl und substituiertem Cycloalakylalkyl darstellen,
• oder eine Gruppe der Formel (R₃₂): 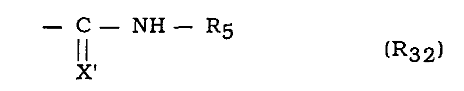 in der X' Schwefel oder Sauerstoff und R₅ Wasserstoff oder eine Gruppe ausgewählt aus Alkyl, substituiertem Alkyl, Cycloalkyl, substituiertem Cycloalkyl, Cycloalkylalkyl und substituiertem Cycloalkylalkyl darstellen,
mit der Maßgabe bedeuten, daß bezüglich der Formel (I) dann, wenn nichts anderes angegeben ist:
- die Begriffe "Alkyl" und "Alkoxy" für geradkettige oder verzweigte Gruppen stehen, die 1 bis 6 Kohlenstoffatome enthalten,
- die Begriffe "Alkenyl" und "Alkinyl" für geradkettige oder verzweigte Gruppen stehen, die 2 bis 6 Kohlenstoffatome enthalten,
- der Begriff "Cycloalkyl" für eine Gruppe mit 3 bis 8 Kohlenstoffatomen steht,
- der Begriff "substituiert" in Verbindung mit einerAlkylgruppe bedeutet, daß diese Gruppe durch einen oder mehrere Substituenten ausgewählt aus Halogen, Alkyl, Hydroxy und Alkoxy substituiert ist,
- der Begriff "substituiert" in Verbindung mit einer "Cycloalkylgruppe" und einer "Cycloalkylalkylgruppe" bedeutet, daß diese Gruppe durch einen oder mehrere Reste oder Gruppen ausgewählt aus Halogen, Alkyl und Oxo substituiert ist,
- der Begriff "substituiert" in Verbindung mit den Begriffen "Indol" und "Indolin" bedeutet, daß diese Gruppen am Stickstoffatom in der 1-Stellung durch eine Gruppe ausgewählt aus -Ra, -CO-Ra und -CO-O-Ra substituiert sind, in der Ra eine Alkyl-, Phenyl- oder Phenylalkylgruppe darstellt,
und deren Enantiomere und Diastereoisomere.

2. Verbindungen der Formel (I) nach Anspruch 1, in der getrennt oder gemeinsam betrachtet:
- R₁ eine Alkylgruppe darstellt,
- R₁ eine (C₂-C₆)-Alkylgruppe darstellt,
- R₁ eine Ethylgruppe darstellt,
- R₁ eine Propylgruppe darstellt,
- R₁ eine Butylgruppe darstellt,
- A zusammen mit dem Benzolkern, an den es gebunden ist, eine Tetrahydronaphthalingruppe bildet,
- Azusammen mit dem Benzolkern, an den es gebunden ist, eine Naphthalingruppe bildet,
- A zusammen mit dem Benzolkern, an den es gebunden ist, eine Dihydronaphthalingruppe bildet,
- A zusammen mit dem Benzolkern, an den es gebunden ist, eine Benzothiophengruppe bildet,
- A zusammen mit dem Benzolkern, an den es gebunden ist, eine Indolgruppe bildet,
- A zusammen mit dem Benzolkern, an den es gebunden ist, eine substituierte Indolgruppe bildet,
- R₂ ein Wasserstoffatom bedeutet,
- R₂ eine Alkylgruppe bedeutet,
- R₃ eine Gruppe R₃₁ bedeutet, wie sie bezüglich der Formel (I) definiert ist,
- R₃ eine Gruppe R₃₂ bedeutet, wie sie bezüglich der Formel (I) definiert ist,
- R₄ ein Wasserstoffatom bedeutet,
- R₄ eine Alkylgruppe bedeutet,
- R₄ eine Cycloalkylgruppe bedeutet,
- R₄ eine Alkenylgruppe bedeutet,
- R₅ ein Wasserstoffatom bedeutet,
- R₅ eine Alkylgruppe bedeutet,
- R₅ eine Cycloalkylgruppe bedeutet,
- X ein Sauerstoffatom bedeutet,
- X ein Schwefelatom bedeutet,
- X' ein Sauerstoffatom bedeutet,
- oder X' ein Schwefelatom bedeutet.

3. Verbindungen der Formel (I) nach Anspruch 1 entsprechend den folgenden Formeln (1) bis (5):

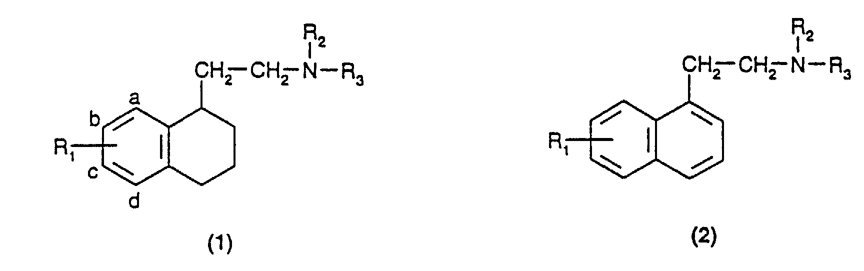

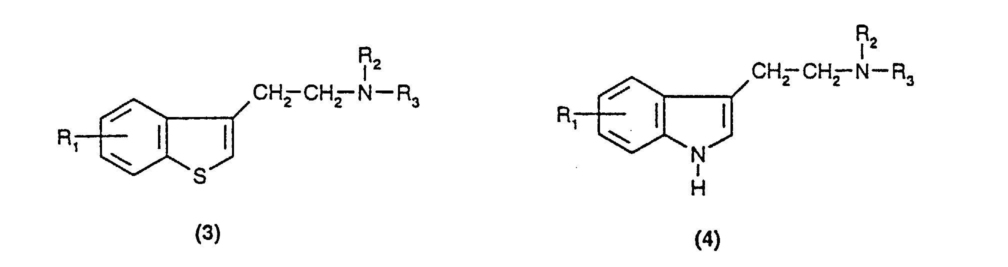

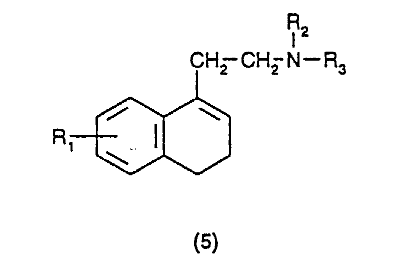

4. Verbindungen nach Anspruch 1, nämlich:
- N-[2-(5-Ethyl-benzothiophen-3-yl)-ethyl]-acetamid,
- N-[2-(5-Ethyl-benzothiophen-3-yl)-ethyl]-cyclobutancarboxamid,
- N-[2-(7-Ethyl-1,2,3,4-tetrahydronaphth-1-yl)-ethyl]-acetamid,
- N-[2-(7-Ethyl-1,2,3,4-tetrahydronaphth-1-yl)-ethyl]-butyramid,
- N-[2-(7-Ethyl-1,2,3,4-tetrahydronaphth-1-yl)-ethyl]-cyclopropancarboxamid,
- N-[2-(7-Ethyl-1,2,3,4-tetrahydronaphth-1-yl)-ethyl]-pentanamid,
- und N-[2-(7-Ethyl-1,2,3,4-tetrahydronaphth-1-yl)-ethyl]-trifluoracetamid.

5. Verfahren zur Herstellung der Verbindungen der Formel (I), dadurch gekennzeichnet, daß man eine Verbindung der Formel (II): 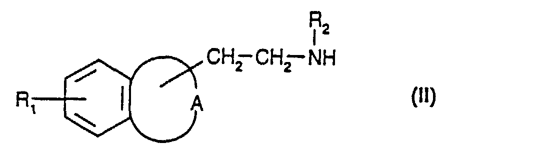 in der R₁ und A die in Anspruch 1 angegebenen Bedeutungen besitzen,
- entweder mit Ameisensäure oder mit einer Verbindung der Formel (IIIa) oder (IIIb): 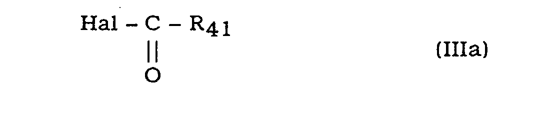 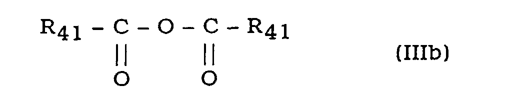 worin R₄₁ die in Anspruch 1 angegebenen Bedeutungen besitzt und Hal ein Halogen darstellt,
umsetzt, so daß man die Verbindungen der Formel (I/a) erhält: 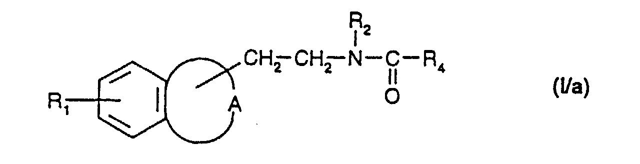 in der R₁, R₂, R₄ und A die oben angegebenen Bedeutungen besitzen,
welche Verbindungen der Formel (I/a) mit dem Lawesson-Reagens umgesetzt werden zur Bildung der Verbindungen der Formel (I/a'): 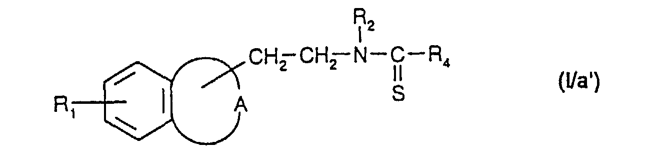 in der R₁, R₂, R₄ und A die oben angegebenen Bedeutungen besitzen,
- oder mit einer Verbindung der Formel (IV):
X' = C = N - R₅ (IV)
in der X' und R₅ die in Anspruch 1 angegebenen Bedeutungen besitzen, umsetzt zur Bildung der Verbindungen der Formel (I/b): 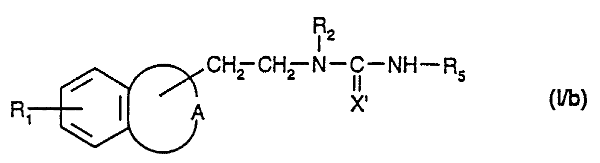 in der R₁, R₂, R₅, A und X' die oben angegebenen Bedeutungen besitzen, welche Verbindungen der Formeln (I/a), (I/a') und (I/b) die Gesamtheit der Verbindungen der Formel (I) nach Anspruch 1 bilden, welche Verbindungen der Formel (I) gegebenenfalls in ihre verschiedenen Enantiomeren oder Diastereoisomeren aufgetrennt werden.

6. Verfahren nach Anspruch 5 zur Herstellung der Verbindungen der Formel (I/c): 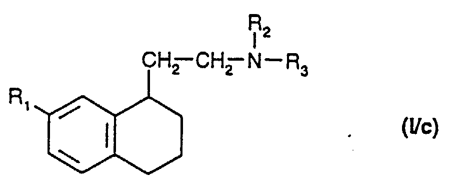 in der R₁, R₂ und R₃ die in Anspruch 1 angegebenen Bedeutungen besitzen, dadurch gekennzeichnet, daß man:
eine Verbindung der Formel (II/a): 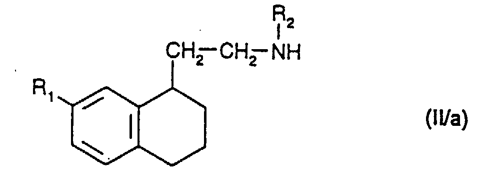 in der R₁ und R₂ die oben angegebenen Bedeutungen besitzen,
- entweder mit einer Verbindung der Formel (IIIa) oder (IIIb), wie sie in Anspruch 5 definiert ist, umsetzt,
zur Bildung der Verbindungen der Formel (I/d): 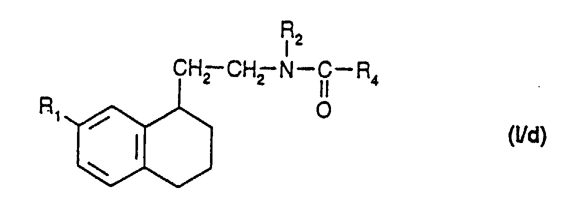 in der R₁, R₂ und R₄ die oben angegebenen Bedeutungen besitzen, welche anschließend mit dem Lawesson-Reagens umgesetzt werden zur Bildung der Verbindungen der Formel (I/d'): 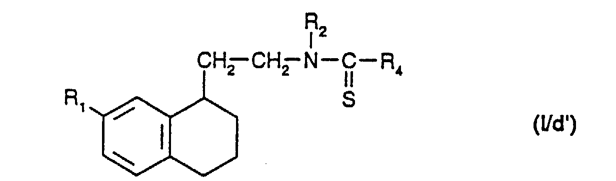 in der R₁, R₂ und R₄ die oben angegebenen Bedeutungen besitzen,
- oder mit einer Verbindung der Formel (IV), wie sie oben definiert worden ist, umsetzt,
zur Bildung der Verbindungen der Formel (I/e): 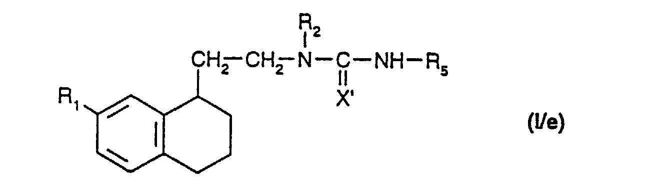 in der R₁, R₂, R₅ und X' die oben angegebenen Bedeutungen besitzen, welche Verbindungen der Formeln (I/d), (I/d') und (I/e) die Gesamtheit der Verbindungen der Formel (I/c) bilden,
welche Verbindungen der Formel (I/c) in ihre verschiedenen Enantiomeren oder Diastereoisomeren aufgetrennt werden können.

7. Verfahren zur Herstellung der Verbindungen der Formel (I/f): 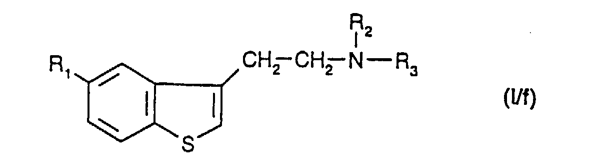 in der R₁, R₂ und R₃ die in Anspruch 1 angegebenen Bedeutungen besitzen,
dadurch gekennzeichnet, daß man:
eine Verbindung der Formel (II/b): 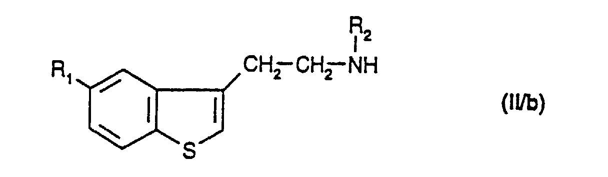 in der R₁ und R₂ die oben angegebenen Bedeutungen besitzen,
- entweder mit einer Verbindung der Formel (IIIa) oder (IIIb), wie sie in Anspruch 5 definiert sind, umsetzt,
zur Bildung der Verbindungen der Formel (I/g): 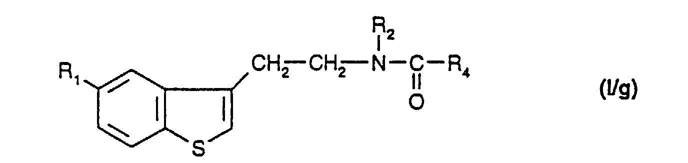 in der R₁, R₂ und R₄ die oben angegebenen Bedeutungen besitzen, welche anschließend mit dem Lawesson-Reagens umgesetzt werden zur Bildung der Verbindungen der Formel (I/g'): 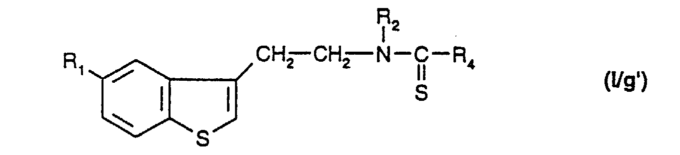 in der R₁, R₂ und R₄ die oben angegebenen Bedeutungen besitzen,
- oder mit einer Verbindung der Formel (IV), wie sie oben definiert worden ist, umsetzt, zur Bildung der Verbindungen der Formel (I/h): 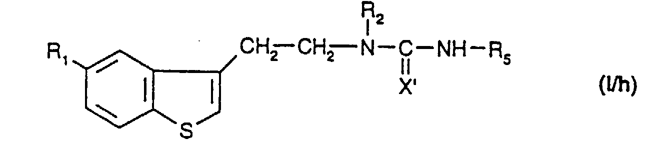 in der R₁, R₂, R₅ und X' die oben angegebenen Bedeutungen besitzen,
welche Verbindungen der Formeln (I/g), (I/g') und (I/h) die Gesamtheit der Verbindungen der Formel (I/f) bilden,
welche Verbindungen der Formel (I/f) in ihre verschiedenen Enantiomeren oder Diastereoisomeren aufgetrennt werden können.

8. Pharmazeutische Zubereitungen enthaltend Produkte der Formel (I) nach Anspruch 1 in Kombination mit einem oder mehreren pharmazeutisch annehmbaren Trägermaterialien.

9. Pharmazeutische Zubereitungen nach Anspruch 8 zur Behandlung von melatoninergischen Störungen.

10. Pharmazeutische Zubereitungen nach Anspruch 8 zur Behandlung von jahreszeitlich bedingten Depressionen, Schlafstörungen, kardiovaskulären patholgischen Zuständen, Schlaflosigkeit und Ermüdung als Folge von Zeltverschiebungen, Appetitstörungen und Fettsucht.

## Claims

1. Compounds of formula (I): 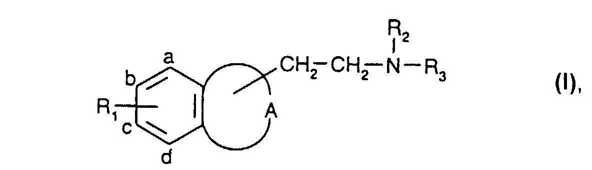 wherein :
- R₁ represents a radical selected from alkyl, substituted alkyl, cycloalkyl, substituted cycloalkyl, cycloalkylalkyl and substituted cycloalkylalkyl,
- A, together with the benzene nucleus to which it is bonded, forms a cyclic group selected from tetrahydronaphthalene, dihydronaphthalene, naphthalene, benzothiophene, 2,3-dihydrobenzothiophene, indoline, substituted indoline, indole and substituted indole,
- R₂ represents a hydrogen atom or an alkyl group,
- R₃ represents :
• a group R₃₁ : 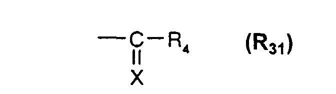 wherein X represents a sulphur atom or an oxygen atom and R₄ represents a hydrogen atom or a radical R₄₁ selected from alkyl, substituted alkyl, alkenyl, alkynyl, cycloalkyl, substituted cycloalkyl, cycloalkylalkyl and substituted cycloalkylalkyl,
• or a group of formula (R₃₂) : 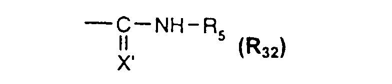 wherein X' represents a sulphur atom or an oxygen atom and R₅ represents a hydrogen atom or a radical selected from alkyl, substituted alkyl, cycloalkyl, substituted cycloalkyl, cycloalkylalkyl and substituted cycloalkylalkyl,
it being understood that, in the description of formula (I) and unless specified otherwise :
- the terms "alkyl" and "alkoxy" denote linear or branched groups containing from 1 to 6 carbon atoms,
- the terms "alkenyl" and "alkynyl" denote linear or branched groups containing from 2 to 6 carbon atoms,
- the term "cycloalkyl" denotes a group having from 3 to 8 carbon atoms,
- the term "substituted" associated with an alkyl radical indicates that such a radical is substituted by one or more substituents selected from halogen, alkyl, hydroxy and alkoxy,
- the term "substituted" associated with a cycloalkyl or cycloalkylalkyl radical indicates that such a radical is substituted by one or more radicals or groups selected from halogen, alkyl and oxo,
- the term "substituted" associated with the terms "indole" and "indoline" indicates that such groups are substituted at the nitrogen in the 1-position by a radical selected from -Ra, -CO-Ra and -CO-O-Ra, wherein Ra represents an alkyl, phenyl or phenylalkyl radical,
and enantiomers and diastereoisomers thereof.

2. Compounds of formula (I) according to claim 1 wherein, taken separately or together,
- R₁ represents an alkyl group,
- R₁ represents a (C₂-C₆)alkyl group,
- R₁ represents an ethyl group,
- R₁ represents a propyl group,
- R₁ represents a butyl group,
- A, together with the benzene nucleus to which it is bonded, forms a tetrahydronaphthalene group,
- A, together with the benzene nucleus to which it is bonded, forms a naphthalene group,
- A, together with the benzene nucleus to which it is bonded, forms a dihydronaphthalene group,
- A, together with the benzene nucleus to which it is bonded, forms a benzothiophene group,
- A, together with the benzene nucleus to which it is bonded, forms an indole group,
- A, together with the benzene nucleus to which it is bonded, forms a substituted indole group,
- R₂ represents a hydrogen atom,
- R₂ represents an alkyl group,
- R₃ represents a group R₃₁ as defined for formula (I),
- R₃ represents a group R₃₂ as defined for formula (I),
- R₄ represents a hydrogen atom,
- R₄ represents an alkyl group,
- R₄ represents a cycloalkyl group,
- R₄ represents an alkenyl group,
- R₅ represents a hydrogen atom,
- R₅ represents an alkyl group,
- R₅ represents a cycloalkyl group,
- X represents an oxygen atom,
- X represents a sulphur atom,
- X' represents an oxygen atom,
- or X' represents a sulphur atom.

3. Compounds of formula (I) according to claim 1 corresponding to the respective formulae (1) to (5) :

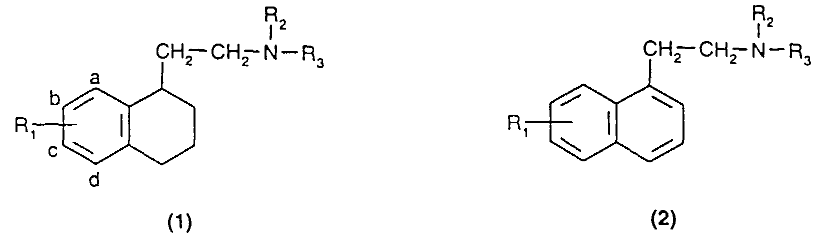

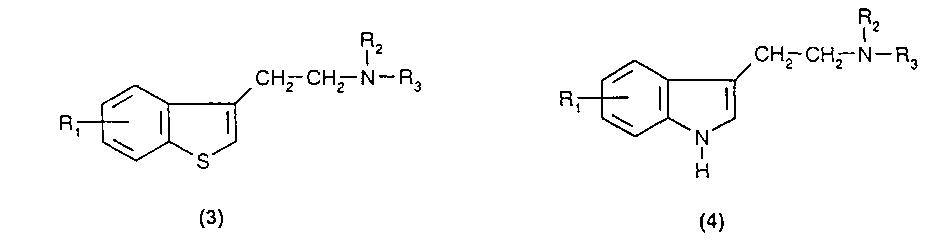

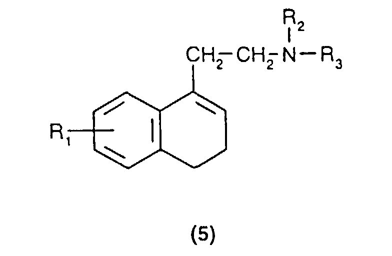

4. Compounds according to claim 1 which are:
- N-[2-(5-ethyl-benzothiophen-3-yl)ethyl]acetamide,
- N-[2-(5-ethyl-benzothiophen-3-yl)ethyl]cyclobutanecarboxamide,
- N-[2-(7-ethyl-1,2,3,4-tetrahydronaphth-1-yl)ethyl]acetamide,
- N-[2-(7-ethyl-1,2,3,4-tetrahydronaphth-1-yl)ethyl]butyramide,
- N-[2-(7-ethyl-1,2,3,4-tetrahydronaphth-1-yl)ethyl]cyclopropanecarboxamide,
- N-[2-(7-ethyl-1,2,3,4-tetrahydronaphth-1-yl)ethyl]pentanamide
- and N-[2-(7-ethyl-1,2, 3,4-tetrahydronaphth-1-yl)ethyl]trifluoroacetamide.

5. Process for the preparation of compounds of formula (I), characterised in that: a compound of formula (II) : 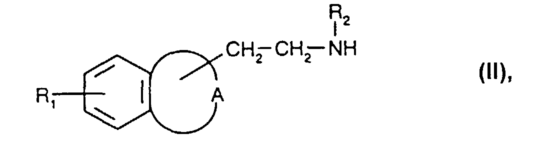 wherein R₁ and A are as defined in claim 1,
is reacted
- either with formic acid or with a compound of formula (IIIa) or (IIIb) : 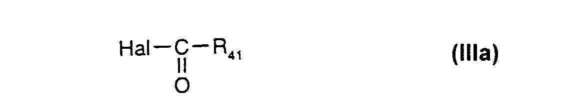 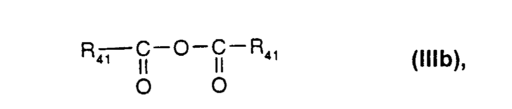 wherein R₄₁ is as defined in claim 1 and Hal represents a halogen atom, to obtain the compounds of formula (I/a): 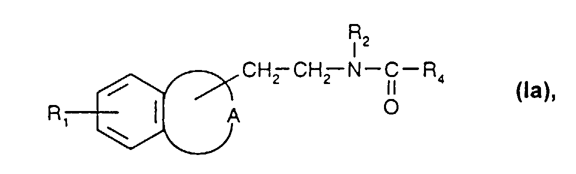 wherein R₁, R₂, R₄ and A are as defined hereinbefore,
which compounds of formula (la) are treated with Lawesson's reagent to obtain the compounds of formula (I/a'): 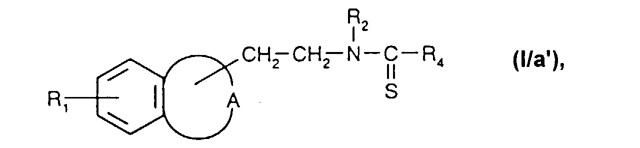 wherein R₁, R₂, R₄ and A are as defined hereinbefore,
- or with a compound of formula (IV) :
X'=C=N-R₅ **(IV),**
wherein X' and R₅ are as defined in claim 1,
to obtain the compounds of formula (I/b) : 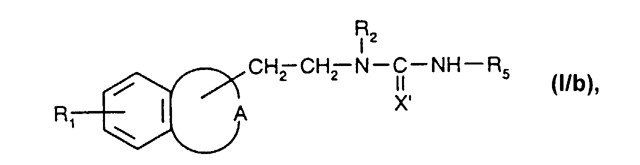 wherein R₁, R₂, R₅, A and X' are as defined hereinbefore,
the compounds of formulae (I/a), (I/a') and (I/b) constituting the totality of the compounds of formula (I) according to claim 1, which compounds of formula (I), if necessary, are separated into their different enantiomers or diastereoisomers.

6. Process according to claim 5 for the preparation of compounds of formula (I/c): 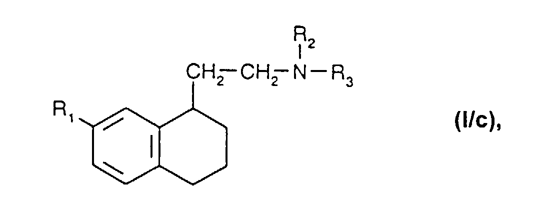 wherein R₁, R₂ and R₃ are as defined in claim 1,
characterised in that : a compound of formula (II/a) : 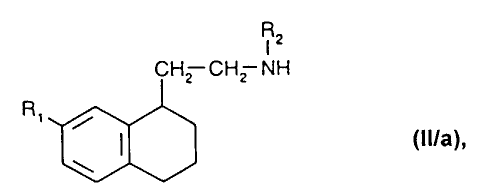 wherein R₁ and R₂ are as defined hereinbefore,
is reacted
- either with a compound of formula (IIIa) or (IIIb) as defined in claim 5, to obtain the compounds of formula (I/d) : 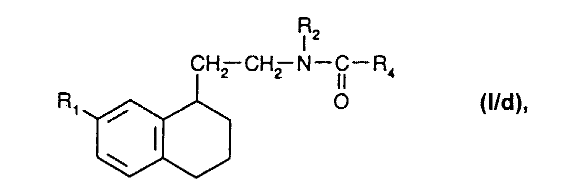 wherein R₁, R₂ and R₄ are as defined hereinbefore, which are then treated with Lawesson's reagent to obtain the compounds of formula (I/d') : 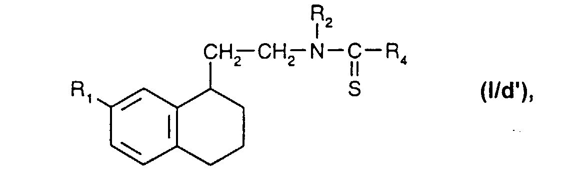 wherein R₁, R₂ and R₄ are as defined hereinbefore,
- or with a compound of formula (IV) as defined hereinbefore,
to obtain the compounds of formula (I/e) : 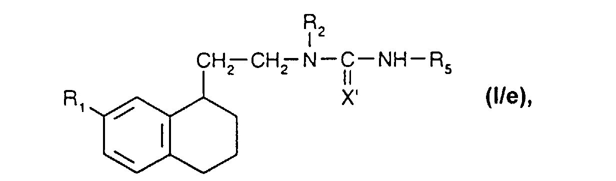 wherein R₁, R₂, R₅ and X' are as defined hereinbefore,
the compounds of formulae (I/d), (I/d') and (I/e) constituting the totality of the compounds of formula (I/c),
which compounds of formula (I/c) may be separated into their different enantiomers or diastereoisomers.

7. Process for the preparation of compounds of formula (I/f) : 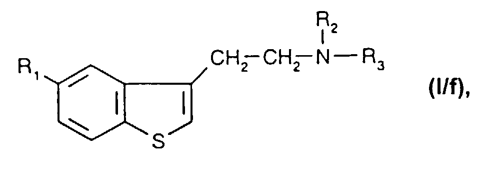 wherein R₁, R₂ and R₃ are as defined in claim 1,
characterised in that :
a compound of formula (II/b): 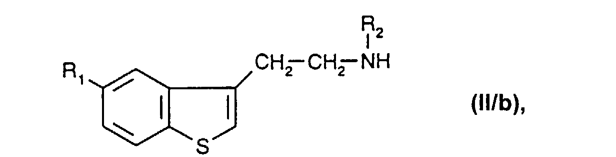 wherein R₁ and R₂ are as defined hereinbefore,
is reacted
- either with a compound of formula (IIIa) or (IIIb) as defined in claim 5, to obtain the compounds of formula (I/g): 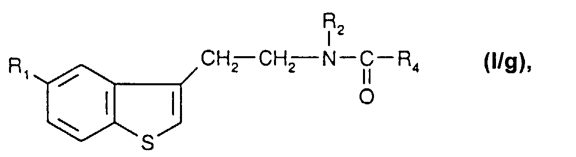 wherein R₁, R₂ and R₄ are as defined hereinbefore, which are then treated with Lawesson's reagent to obtain the compounds of formula (I/g'): 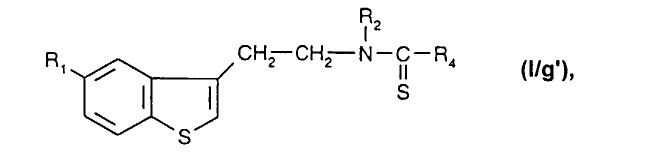 wherein R₁, R₂ and R₄ are as defined hereinbefore,
- or with a compound of formula (IV) as defined hereinbefore, to obtain the compounds of formula (I/h): 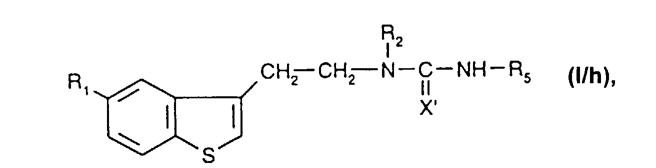 wherein R₁, R₂, R₅ and X' are as defined hereinbefore,
the compounds of formulae (I/g), (I/g') and (I/h) constituting the totality of the compounds of formula (I/f),
which compounds of formula (I/f) may be separated into their different enantiomers or diastereoisomers.

8. Pharmaceutical compositions comprising the compounds of formula (I) according to claim 1 in combination with one or more pharmaceutically acceptable excipients.

9. Pharmaceutical compositions according to claim 8 for use in the treatment of melatoninergic disorders.

10. Pharmaceutical compositions according to claim 8 for use in the treatment of seasonal affective disorders, sleep disorders, cardiovascular pathologies, insomnia and fatigue due to jetlag, appetite disorders and obesity.
